# EUROPÄISCHE PATENTANMELDUNG

(11) **EP 0 939 125 A2**
(43) Veröffentlichungstag der Anmeldung: **01.09.1999**
(21) Anmeldenummer: 99102793.9
(22) Anmeldetag: 24.02.1999
(51) Int. Cl.: C12N 15/12, C12N 15/54, C12N 15/63, C12N 15/85, C12N 5/10, C12N 9/12, C07K 14/82, C07K 14/47

(54) **Expressionsvektor zur konditionalen Regulation der Expression der grossen Untereinheit der RNA-Polymerase II**

(30) Priorität: 27.02.1998 DE 19808453
(71) Anmelder: GSF-Forschungszentrum für Umwelt und Gesundheit, GmbH, 85764 Oberschleissheim (DE)
(72) Erfinder: Eick, Dirk, 82110 Germering (DE); Meininghaus, Mark, 80339 München (DE)
(74) Vertreter: Behnisch, Werner, Dr.

(57) **Zusammenfassung**

Erfindungsgemäß wird ein Expressionsvektor für Eukaryontenzellen bereitgestellt, der zumindest die nachfolgenden Elemente enthält:
(a) Das Gen für die große Untereinheit der RNA-Polymerase II oder mutierten Formen davon, wobei die RNA-Polymerase II oder die mutierten Formen davon gegen α-Amanitin oder einem Derivat hiervon resistent sind;
(b) Nukleotidsequenzen, die eine konditionale Regulation der Expression der großen Untereinheit des α-Amanitin-resistenten RNA-Polymerase II-Gens oder der mutierten Formen ermöglichen;
(c) Nukleotidsequenzen zur Selektion des Vektors in Prokaryonten- und/oder Eukaryonten-Zellen;
(d) Nukleotidsequenzen zur Replikation des Vektors in Prokaryonten-Zellen und/oder Eukaryonten-Zellen;
(e) eine Polyadenylierungsstelle (PAA).

## Beschreibung

Die Erfindung betrifft einen Expressionsvektor für Eukaryontenzellen, Verwendungen dieses Expressionsvektors sowie ein Verfahren zur konditionalen, stabilen Expression von α-Amanitinresistenter RNA-Polymerase II unter Verwendung des Expressionsvektors.

Das menschliche Genom kodiert nach Schätzungen für ca. 100 000 unterschiedliche Gene. Mehr als 99% dieser Gene werden von der RNA Polymerase II (Pol II) transkribiert. Die Entwicklung von Organismen und die Differenzierung von Zeilen wird vornehmlich durch differenzielle Expression von Genen gesteuert, die durch RNA Polymerase II abgelesen werden. Die Anzahl der Pol I und Pol III transkribierten Genen ist dagegen gering. Pol II ist nicht in der Lage, spezifische DNA Sequenzen zu erkennen und selbst an DNA zu binden. Die Polymerase benötigt dazu Hilfsfaktoren, die basale Transkriptionsfaktoren genannt werden. Nach Bindung von Pol II an die DNA mit Hilfe von basalen Transkriptionsfaktoren ist die erfolgreiche Transkription eines Gens in der Regel noch nicht möglich. Pol II benötigt weitere Unterstützung durch sogenannte Transkriptionsaktivatoren, um Transkriptionsaktivität zu erlangen.

Pol II ist, änhlich wie Pol I und Pol III, aus über 10 Untereinheiten aufgebaut. Die großen Untereinheiten sind zwischen den einzelnen Polymerasen sehr ähnlich, einige kleinere Untereinheiten sind sogar identisch, andere Untereinheiten unterscheiden sich deutlich. Pol II unterscheidet sich von Pol I und Pol III durch ein wesentliches Detail. Die große Untereinheit von Pol II besitzt zusätzlich eine hochrepetitive carboxy-terminale-Domäne(CTD). Die CTD besteht bei Maus und Mensch aus 52 Repeats eines Hepta-Peptids mit der Konsensussequenz Ser-Pro-Thr-Ser-Pro-Ser-Tyr. Die große Untereinheit von Pol II in der Hefe besitzt eine sehr ähnliche Struktur, allerdings findet man nur 26 Repeats (in Drosophila 42 Repeats). Es fällt auf, daß fünf der sieben Aminosäuren eines Hepta-Peptids potentiell phosphoryliert werden können. Tatsächlich findet man Pol II in zwei Phosphorylierungszuständen in der Zelle vor, die vor allem auf eine unterschiedliche Phosphorylierung der CTD zurückzuführen sind. Die hypophosphorylierte Form wird Pol IIa, die hyper-phosphorylierte Form Pol IIO genannt. Man geht davon aus, daß nur Pol IIa an einen Promotor bindet und daß der Übergang zur Pol IIO erst nach Beginn des Transkriptionprozesses erfolgt.

Tatsächlich konnten wir und andere zeigen, daß Pol II in vivo nach Bindung an Promotoren weitere Aktivierungssignale benötigt, um in einen prozessiven Transkriptionsmodus überführt zu werden. Eine große Anzahl von Experimenten deutet darauf hin, daß dieser Aktivierungsschritt mit der Phosphorylierung von CTD in Zusammenhang steht. Eine Anzahl von Kinasen ist in der Zwischenzeit beschrieben worden, die CTD in vitro phosphorylieren können. Ob diese Kinasen CTD auch in vivo phosphorylieren ist unklar. Weiter ist heute vollkommen unklar, welche Phosphorylierungsmuster Kinasen in der CTD einführen müssen, um Pol II zu aktivieren. Im Extremfall wäre es denkbar, daß Pol II für die Transkription jedes individuellen Gens ein spezifisches CTD-Phosphorylierungsmuster benötigt. In diesem Fall würden wahrscheinlich die Aktivitäten von unterschiedlichen Signalketten in der CTD integriert und daraufhin in Genaktivität umgesetzt. Das extreme Gegenteil von dieser Sichtweise ist die Annahme, daß durch CTD-Phosphorylierung Genaktivität nicht individuell gesteuert werden kann. In diesem Fall würde wahrscheinlich immer ein sehr ähnliches Phosphorylierungsmuster in CTD eingeführt. Diese Annahme ist aber eher unwahrscheinlich, wie neue Experimente aus unserem Labor zeigen. Eine ganz zentrale Frage in diesem Zusammenhang ist aber, wie die Struktur der CTD in vivo aussieht und welche molekularen Vorgänge nach der Phosphorylierung der CTD zur Aktivierung der Pol II führen.

Pol I, II und III sind unterschiedlich sensitiv gegenüber α-Amanitin, einem Gift aus dem Knollenblätterpilz. Pol II wird bereits bei Konzentrationen gehemmt, bei denen Pol I und Pol III noch voll ihre Funktion ausüben. Von Jeffry Corden und Mitarbeitern wurde vor zehn Jahren eine Pol II Mutante isoliert, die α-Amanitin-resistent ist. Die Resistenz beruht auf einer Punktmutation, die nicht in der großen Untereinheit der CTD liegt. Zellen mit dieser Punktmutation können in Gegenwart von α-Amanitin wachsen. Corden und Mitarbeiter sowie mehrere andere Arbeitsgruppen haben Untersuchungen zur Funktion der CTD durchgeführt. Übereinstimmend konnte für verschiedene Organismen gezeigt werden, daß die Verkürzung der CTD unter eine kritische Anzahl von Repeats zum Tod einer Zelle führt. Der Zelltod ist sehr wahrscheinlich darauf zurückzuführen, daß bestimmte Gene nicht mehr exprimiert werden können.

Die bisher vorgenommenen Untersuchungen an Mutanten der RNA-Polymerase II umfaßten ausschließlich transiente Transfektionsexperimente, die unter anderem die nachfolgenden Nachteile aufweisen:
(i) Nur ein Bruchteil aller Zellen wird in einer Transfektion erreicht; nicht transfizierte Zellen erzeugen einen Hintergrund;
(ii) große Plasmidkonstrukte wie hier beschrieben mit >25 kb werden nur sehr ineffizient aufgenommen;
(iii) Zellen sind nicht in beliebiger Größenordnung transfizierbar;
(iv) DNA Transfektion erzeugt Streß in Zellen und führt zu nicht gewünschten Nebenreaktionen, wie z. B. Zellzyklusarrest durch Induktion des p53 Gens;
(v) die Menge der aufgenommenen Plasmid DNA ist nicht gut steuerbar;
(vi) die Menge des exprimierten Genprodukts ist nicht gut steuerbar;
(vii) das transfizierte Gen läßt sich nicht wieder abstellen;
(viii) die generelle Reproduzierbarkeit der Experimente ist eingeschränkt durch die Vielzahl der Transfektionsparameter;
(ix) aufgrund der oben angeführten Gründe konnte der Phänotyp für α-Amanitin-resistente LS-Pol II Muntanten bisher nur mit Hilfe von co-transfizierten Reportergenen in transienten Transfektionsexperimenten untersucht werden. Untersuchungen, welchen Phänotyp LS-Pol II Muntanten für die Expression chromosomaler Genen verursachen, konnten bisher nicht durchgeführt werden.

Die Mutagenese von Genen ist die Standardtechnik, um die Struktur und Funktion von Proteinen aufzuklären. Die Technik ist vor allem dann sehr erfolgreich, wenn das veränderte Gen die Lebensfähigkeit einer Zelle oder eines Organismus nicht nachhaltig beeinträchtigt.

Die große Untereinheit der RNA Polymerase II (LS-Pol II) ist zentral für die Funktion einer eukaryonten Zelle. Eine Vielzahl von LS-Pol II Mutanten ist letal und kann daher nicht in Zell-Linien untersucht werden, die diese Mutante stabil exprimieren.

Es ist daher eine Aufgabe der vorliegenden Erfindung, einen Expressionsvektor für Eukaryontenzellen bereit zu stellen, der die oben genannten Nachteile des Standes der Technik vermeidet.

Diese Aufgabe wird erfindungsgemäß durch den Expressionsvektor gemäß Anspruch 1 der beiliegenden Patentansprüche gelöst. Der Expressionsvektor wird für Untersuchungen zur Funktionalität einzelner Domänen des RNA-Polymerase II-Gens und dem Zusammenwirken dieser Domänen mit anderen Proteinen und DNA bevorzugt eingesetzt. Bevorzugte Ausführungsformen des erfindungsgemäßen Vektors, bevorzugte Ausführungsformen der erfindungsgemäßen Verwendung und ein Verfahren zur konditionalen stabilen Expression von der großen Untereinheit α-Amanitin-resistenter RNA-Polymerase II unter Verwendung des erfindungsgemäß bereit gestellten Expressionsvektors werden in den Unteransprüchen beschrieben.

Der erfindungsgemäße Expressionsvektor ist so ausgelegt, daß das Gen für die große Untereinheit der RNA-Polymerase II in Eukaryontenzellen konditional exprimierbar ist. Hierfür sind zumindest die nachfolgenden Elemente im Expressionsvektor enthalten, wobei in einer weiteren Ausführungsform der Erfindung diese Elemente auf zumindest zwei voneinander getrennten Vektoren lokalisiert sind:
(a) Das Gen für die große Untereinheit der RNA-Polymerase II oder mutierte Formen hiervon, mit der Eigenschaft, der RNA-Polymerase II eine Resistenz für α-Amanitin oder einem Derivat hiervon zu vermitteln. Mutierte Formen dieses Gens sind in bevorzugten Ausführungsformen der Erfindung einsetzbar, wobei die Einführung von Mutationen, insbesondere von Deletionen, in die große Untereinheit des Polymerase II-Gens dem Fachmann an sich bekannt ist. Bevorzugt wird für die vorliegenden Untersuchungen eine mutierte Form der großen Untereinheit der RNA-Polymerase II verwendet, die in der carboxy-terminalen Domäne Deletionen der Hepta-Repeats enthält.
   Erfindungsgemäß ist es ausreichend, wenn lediglich die große Untereinheit des RNA-Polymerase II-Gens in exprimierbarer Form im Vektor vorliegt. Die übrigen Untereinheiten der RNA-Polymerase II werden von den endogenen RNA-Polymerase II-Genen bereitgestellt. Die große Untereinheit der RNA-Polymerase II, die vom erfindungsgemäßen Expressionsvektor exprimiert werden kann, ist gegen α-Amanitin resistent. Sie verleiht der gesamten RNA-Polymerase II eine Resistenz gegen α-Amanitin.
(b) Nukleotidsequenzen, die eine konditionale Regulation des Gens der großen Unterheit der α-Amanitin-resistenten RNA-Polymerase II ermöglichen. Hierzu ist insbesondere das von Gossen und Bujard (1992) beschriebene System einsetzbar, das auf der Tetrazyklin (Tc)-abhängigen Bindung des Tet-Repressors (tetR) an eine Operatorsequenz (tetO) basiert. Durch Fusion des tetR mit der Transaktivierungsdomäne des Herpes simplex-Gens VP16 wurde ein Transaktivator (tTA) erzeugt, der in Abwesenheit von Tc an tetO bindet und in der Umgebung befindliche Promotoren aktiviert. In Anwesenheit von Tc wird die Bindung von tTA an tetO aufgehoben und damit die Promotoraktivierung unterbrochen. tetO liegt bevorzugt in 7-facher Kopienzahl vor und wird dann als "tetO7" bezeichnet. Dieses System erfordert für die konditionale Expression des Polymerase II-Gens eine intakte Expressionskassette für tTA sowie eine intakte Transkriptionseinheit für Pol II, bestehend aus einem Promotor, der tetO enthält, dem Leserahmen des Gens und einer Polyadenylierungsstelle (PA).
   Die konditionale Regulation des Gens für die große Untereinheit der α-Amanitin-resistenten RNA-Polymerase II kann erfindungsgemäß auch so gestaltet werden, daß in Anwesenheit von Tetrazyklin das Gen der großen Untereinheit von Pol II abgelesen wird und damit in Abwesenheit von Tetrazyklin die Promotoraktivierung unterbrochen wird.
(c) Nukleotidsequenzen, um eine Selektion des Expressionsvektors der vorliegenden Erfindung in ProkaryontenZellen und/oder Eukaryonten-Zellen zu ermöglichen. Diese Nukleotidsequenzen können beispielsweise für Antibiotikaresistenzgene kodieren. Derartige Selektionsgene sind dem Fachmann an sich bekannt.
(d) Nukleotidsequenzen, die eine Replikation des Expressionsvektors der vorliegenden Erfindung in Prokaryonten-Zellen und/oder in Eukaryonten-Zellen ermöglichen.
(e) Eine Polyadenylierungsstelle (PAA).

Nachfolgend wird die Erfindung anhand von Ausführungsbeispielen und Figuren näher beschrieben. Die Erfindung ist jedoch nicht auf die nachfolgenden Ausführungsbeispiele beschränkt.

Erfindungsgemäß wurden die α-Amanitin-resistente Pol II Mutante und die CTD-Deletionsmutanten in einen Tetracyclin-regulierbaren Expressionsvektor mit dem Gen für Hygromycinresistenz kloniert (Abb. 30). Der hierfür benutzte Vektor wird nachstehend noch näher beschrieben werden.

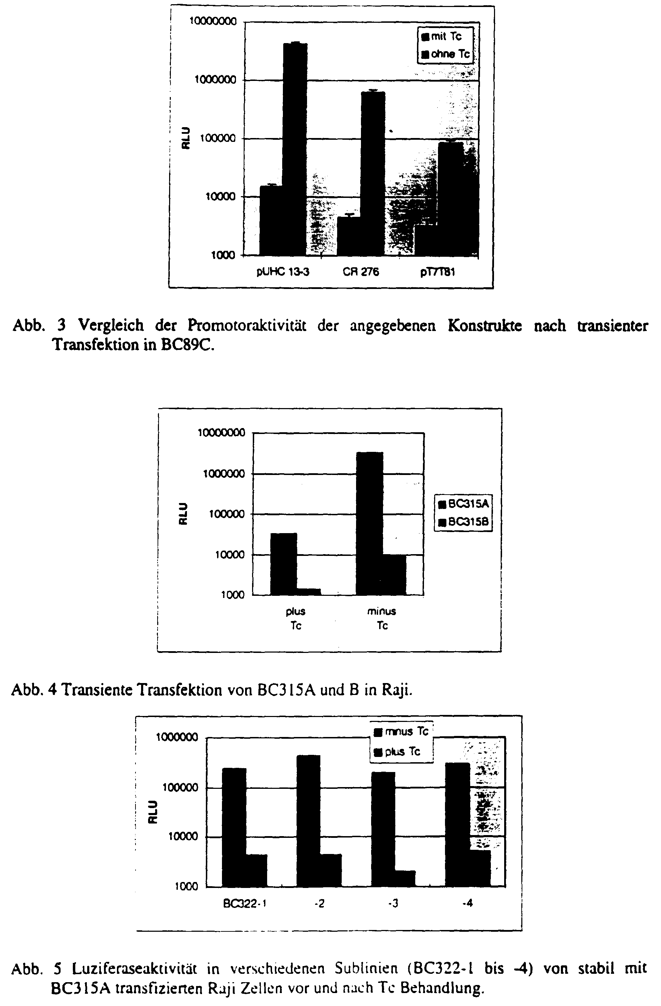

Wir haben zusätzlich eine Variante dieses Vektors konstruiert, die statt des Gens für Hygromycinresistenz das Gen für Neomycinresistenz enthält, so daß nun zwei Vektoren zur Verfügung stehen, die eine Selektion mit Hygromycin oder mit Neomycin ermöglichen (Abbildung 31). In diese Vektoren wurde ein Expressionsgen für die α-Amanitin-resistente, große Untereinheit der Pol II kloniert. Zusätzlich wurden zwei Deletionsformen der CTD kloniert, in denen noch 31(Δ31) und 5(Δ5) Hepta-Repeats vorhanden sind (Abbildung 3). Alle Expressionskonstrukte wurden stabil in eine B-Zell-Linie Raji transfiziert, und resistente Zellpopulationen wurden selektioniert. Alle isolierten Zellpopulationen zeigten keine oder nur eine verschwindend geringe Expression der rekombinanten Pol II. Nach Auswaschen von Tetracyclin wurde in allen Zellpopulationen die rekombinante Pol II > 100-fach induziert (Abbildung 4). Die Proteinmengen der rekombinanten Pol II waren nach 24 h auf einem Niveau exprimiert, ähnlich dem der endogenen Pol II in nicht-α-Amanitin behandelten Zellen (Daten nicht gezeigt). Die Behandlung mit α-Amanitin führt dazu, daß nach 24 h die große Untereinheit der endogenen Pol II nicht mehr nachweisbar ist (Daten nicht gezeigt). Vierundzwanzig Stunden nach Induktion der rekombinanten Pol II erfolgte daher die Zugabe von α-Amanitin, um nach weiteren 24 h einen vollständigen Austausch der endogenen gegen die exogene Pol II Mutante zu erreichen. Zellen, die die α-Amanitin-resistente Form von Pol II mit 52 Hepta-Repeats trugen, waren in ihrer Vitalität nicht wesentlich beeinträchtigt und konnten über mindestens 5 Wochen profilieren. Zellen, die die Δ31 Form exprimieren, proliferierten anfangs, starben aber zwischen Tag 3 und Tag 10. Zellen, die die Δ5 Form exprimierten, starben zwischen Tag 3 und Tag 5.

Die Gene auf dem Vektor sind in Gegenwart von α-Amanitin im allgemeinen nicht exprimierbar, da sie von der α-Amanitin-sensitiven, endogenen RNA-Polymerase II nach Induktion der Expression durch Auswaschen von Tetrazyklin transkribiert werden. Nur dann, wenn vom Vektor nach der Induktion vollständige, d. h. nicht CTD-deletierte Formen der RNA-Polymerase II exprimiert werden, kann die endogene Polymerase auch Gene des Vektors transkribieren. Wird dagegen eine CTD-Mutante vom Vektor exprimiert, kann diese Mutante im allgemeinen keine Vektorgene mehr transkribieren. Zellen haben nach Induktion über einen gewissen Zeitraum, im allgemeinen ca. 24 Stunden, Zeit, nur mRNA zu produzieren, die spezifisch für Vektor-kodierte RNA-Polymerase II ist. Danach wird durch Zugabe von α-Amanitin die Produktion blockiert. Es wurde überraschend gefunden, daß das rekombinante RNA-Polymerase II-Molekül nach Zugabe von α-Amanitin noch 2 Tage nahezu unverändert nachweisbar ist.

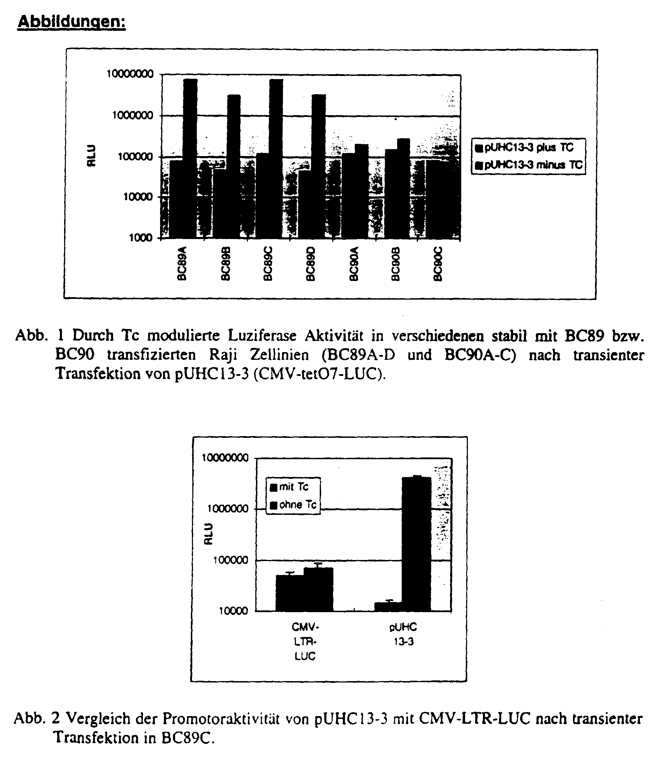

In einer Zelle gibt es eine Vielzahl von Genen, die durch exogene Stimuli innerhalb von Minuten aktiviert werden können. Solche Gene können unmittelbar durch Signalketten induziert werden, ohne daß die Neusynthese von Proteinen benötigt wird. Zu diesen Genen gehören unter anderem das Proto-Onkogen c-fos, das durch Phorbolester und das Heat Shock Gen hsp70, das durch Temperaturen >41°C induziert wird. Wir haben untersucht, ob alle drei Formen der rekombinanten Pol II noch in der Lage sind, das c-fos und hsp70 Gen zu induzieren. Die Pol II mit vollständigem CTD und die Δ31 Mutante konnten beide noch das c-fos und das hsp70 Gen induzieren. Die Mutante Δ5 konnte dagegen keines der beiden Gene mehr induzieren (Abbildung 2, Daten werden nur für hsp 70 gezeigt). Da die CTD für den Transkriptionsvorgang in vitro nicht benötigt wird, sollte die Enzymaktivität der Δ5 Mutante nicht beeinträchtigt sein. Der Defekt der Δ5 Mutante wird auf der Ebene der Initiation und der frühen RNA-Elongation vermutet.

Nachfolgend wird die Erfindung anhand eines Expressionsvektors für Eurkaryontenzellen beschrieben, der episomal repliziert und nicht in die Wirts-DNA integriert. Dieser Vektor enthält Elemente aus dem EBV, beispielsweise aus der EBNA1-Gen. Es ist aber auch möglich, andere, nicht auf EBV-basierende Expressionsvektoren zu konstruieren. Ein Beispiel hierfür ist ein auf BPV-basierender Expressionsvektor. Die EBV-Sequenzen enthaltenden Expressionsvektoren sind in B-Zellen exprimierbar.

### Klonierung der RNA Pol II CTD-Mutanten

**HAwt, HAΔ31, HAΔ5**
   Lit: Gerber-HP et al., Nature 374,660-662(1995)
   **BC252:** vgl. nachfolgende Beschreibung
**pPVETKNE**
   Das in diesem Vektor befindliche Neomycinresistenzgen stammt ursprünglich von Stratagene (XhoI-Sal I-Fragment aus pMC1 neo Poly A).
   Lit: Karreman-SA, Dissertation an der Naturwissenschaftlichen Fakultät der Technischen Universität Carolo-Wilhelmina zu Braunschweig

### Konstruktion von Plasmiden

**MM95:** (ABB. 32)
   In die BglII Schnittstelle von BC252 wurde eine spezielle multiple cloning site (MCS) eingesetzt mit den Schnittstellen: BglII-NotI-SfiI-NgoMI-AscI (MM95).
   Die richtige Orientierung der MCS wurde über Sequenzierung kontrolliert.
**MM 126-10:** (ABB. 33)
   Die Neomycin-Resistenz wurde aus pPVETKNE aus HindIII-BamHI-Fragment (Sequenzposition 2107 bis 3260) mobilisiert und blunt-end in die Restriktionsschnittstellen Tth111I und BstBI (Sequenzposition 5867 bis 7336) des Vektors MM95 einkloniert (MM126-10). Die richtige Orientierung wurde durch geeignete Restriktionsverdaus überprüft (gleiche Orientierung wie HYGr in MM 0951).
**MM 172-3/1:**
   Der vordere Teil des α-Amanitin-resistenten murinen genomischen RNA Polymerase II LS Gens wurde aus HAWt als HaeII-Fragment (Sequenzposition 656 bis 3200) blunt-end in die Restriktionsschnittstelle NotI der MCS des Vektors MM95 eingesetzt (MM172-3/1). Die richtige Orientierung wurde durch geeignete Restriktionsverdaus überprüft.
**MM 172-2/1:**
   Kloniert wie MM172-3/1; nur anstelle von MM95 wurde MM126-10 verwendet.
**MM 217C1:** (ABB. 34)
   Der hintere Teil des α-Amanitin-resistenten murinen genomischen RNA Polymerase II LS Gens mit wt-CTD wurde aus HAwt als SfiI-SfiI-Fragment (Sequenzposition 2744 bis 17282) in die Restriktionsschnittstellen SfiI und SfiI (Sequenzposition 3284 bis 3844) von MM172-3/1 einkloniert.
**MM 217D1:**
   Kloniert wie MM217C1; nur anstelle von HAwt wurde HAΔ31 verwendet.
**MM217e1:**
   Kloniert wie MM217C1; nur anstelle von HAwt wurde HAΔ5 verwendet.
**MM217a2:**
   Der hintere Teil des α-Amanitin-resistenten murinen genomischen RNA Polymerase II LS Gens mit wt-CTD wurde aus HAwt als SfiI-SfiI-Fragment (Sequenzposition 2744 bis 17282) in die Restriktionsschnittstellen SfiI und SfiI (Sequenzposition 3284 bis 3844) von MM172-2/1 einkloniert.
**MM217b1:**
   Kloniert wie MM217a2; nur anstelle von HAwt wurde HAΔ31 verwendet.
**MM203B2:**
   Kloniert wie MM217a2; nur anstelle von HAwt wurde HaΔ5 verwendet.

### Stabile Zellinien

### Verwendete Zellinien

**Parentalzellinie:**
   EBV-positive Burkitt-Lymphom-Linie Raji mit einer t(8; 14)-Translokation.
   Lit: Pulvertaft-RJV Lancet 1,23B-240 (1964)

### Herstellen von Zellinien

**MM 233-2:**
   Die Parentalzellinie Raji wurde stabil mit dem Vektor MM126-10 transfiziert. Die Selektion erfolgte unter G418 und Tetrazyklin.
**MM233-3:**
   Die Parentalzellinie Raji wurde stabil mit dem Vektor MM217a2 transfiziert. Die Selektion erfolgte unter G418 und Tetrazyklin.
**MM233-4:**
   Die Parentalzellinie Raji wurde stabil mit dem Vektor MM217b1 transfiziert. Die Selektion erfolgte unter G418 und Tetrazyklin.
**MM233-6:**
   Die Partentalzellinie Raji wurde stabil mit dem Vektor MM203B2 transfiziert. Die Selektion erfolgte unter G418 und Tetrazyklin.

### Experimente mit den neuen Zellinien

### Testen der Zellinien auf Konditionalität

Um zu testen, ob die RNA Pol II CTD-Mutanten konditional exprimiert werden können, wurde den Zellinien MM233-2, MM233-3, MM233-4 und MM233-6 Tetrazyklin entzogen und eine Western-Blot-Analyse gegen das HA-Epitop der rekombinanten RNA Pol II CTD-Mutanten durchgeführt (siehe Abb. 1).

### Wiederherstellen der hsp70A-Induzierbarkeit unter α-Amanitin:

Für die Induktion der RNA Pol II CTD-Mutanten in den stabilen Zellinien MM233-2 (mock), MM233-3(wt), MM233-4(Δ31) und MM233-6(Δ5) wurde das Tetracyclin entzogen. 24 h danach wurde 2µg/ml α-Amanitin zugesetzt. Weitere 24 h später wurden die Zellen einem Hitzeschock (2h, 43°C) ausgesetzt. In der Northern-Blot-Analyse (geprobt gegen hsp70A, siehe Abb. 2) sieht man, daß wt und Δ31 in der Lage sind, die Induktion des hsp70A-Gens durch Hitzeschock wiederherzustellen. Die CTD-lose Mutante (Δ5) kann dies nicht mehr.

### Wiederherstellen der c-fos-Indzierbarkeit unter α-Amanitin

Für die Induktion der RNA Pol II CTD-Mutanten in den stabilen Zellinien MM233-2 (mock), MM233-3(wt), MM233-4(Δ31) und MM233-6(Δ5) wurde das Tetrazyklin entzogen. 24h danach wurden 2µg/ml α-Amanitin zugesetzt. Weitere 24h später wurden die Zellen mit TPA behandelt. In der Northern-Blot-Analyse (geprobt gegen c-fos, siehe Abb. 3) sieht man, daß wt und Δ31 in der Lage sind, die Induktion des c-fos-Gens durch TPA wiederherzustellen. Die CTD-lose Mutante (Δ5) kann dies nicht mehr.

Unter anderem können die nachfolgenden Fragestellungen durch den erfindungsgemäßen Vektor und das erfindungsgemäße Verfahren analysiert werden:
1. Ist die Expression eines Gens von der CTD in vivo abhängig? Für eine große Anzahl von Genen, die in unserem Zellsystem exprimiert sind, könnte diese Frage sehr einfach beanwortet werden. Unser System könnte unter anderem dazu dienen, Genklassen nach diesem Kriterium zu definieren.
2. Wird eine bestimmte Größe der CTD (Anzahl von Repeats) für die Expression eines bestimmten Gens benötigt?
3. Kann die Expression bestimmter Gene durch gezielte Mutationen in der CTD unterdrückt werden?
4. Für viele Gene ist bekannt, daß sie durch unterschiedliche Stimuli aktiviert werden können. Unklar ist, ob diese verschiedenen Stimuli zu unterschiedlichen CTD-Phosphorylierungsmustern führen, oder umgekehrt, ob z. B. bestimmte CTD-Mutanten das c-fos Gen noch aktivieren können, andere aber nicht mehr.
5. Viele Gene besitzen promotor-proximal eine pausierende Pol II. Kann eine Polymerase ohne CTD bis zu einer Pausierungsstelle transkribieren?
6. Gibt es Kinaseeinhibitoren, die gleichzeitig CTD-Phosphorylierung und RNA-Elongation spezifisch hemmen?
7. Ist die CTD-(De)-Phosphorylierung das Ende der verschiedenen Signalkettenwege oder gibt es CTD-unabhängige Signalwege, die zu einer Transkriptionsaktivierungs/repression führen?

Das oben beschriebene Expressionssystem erlaubt es jetzt erstmals, den Phänotyp aller möglichen LS-Pol II Muntanten zu untersuchen. Diese Untersuchungen werden vor allem auch deswegen möglich, da durch α-Amanitin die endogene Pol II nicht nur in ihrer Aktivität gehemmt wird, sondern diese Hemmung auch zu einem Abbau der endogenen Pol II führt. Bereits 24 h nach Zugabe von α-Amanitin kann die endogene LS-Pol II nicht mehr nachgewiesen werden. Die rekombinante, α-Amanitin-resistente LS-Pol II ist dagegen sehr stabil und wird nach Induktion auch in Gegenwart von α-Amanitin für 48 h nahezu unverändert exprimiert. Dieser Zeitraum reicht aus, um den Einfluß von LS-Pol II Mutanten auf die Expression jedes zellulären Gens zu untersuchen.

Erfindungsgemäß wird somit ein Expressionsvektor bereit gestellt, der die Schaffung stabiler Zell-Linien ermöglicht, die eine α-Amanitin-resistente Form der großen Untereineit des RNA-Polymerase II konditional exprimieren können. Völlig überraschend war, daß auch mutante Formen der großen Untereinheit der RNA-Polymerase II stabil über einen Zeitraum von mindestens 2 Tagen in vitro exprimierbar sind. So wurde beispielsweise festgestellt, daß die Δ5 Mutante für ca. 2 Tage in Gegenwart von α-Amanitin ähnlich gut wie die Δ31 Mutante und die Pol II mit 52 Hepta-Repeats exprimiert wird. Dies bedeutet, daß die Deletion der CTD die Stabilität der rekombinanten RNA-Polymerase II nicht stark herabsetzt, also im erfindungsgemäß bereit gestellten Expressionsvektor in hervorragender und nicht voraussehbarer Weise auch Mutanten der großen Untereinheit der RNA-Polymerase II exprimierbar sind, in denen zumindest ein Teil der Hepta-Repeats deletiert ist.

Nachfolgend werden die erfindungsgemaß bevorzugt verwendbaren Vektoren näher beschrieben.

Erfindungsgemäß wird bevorzugt ein in Eukaryonten episomal replizierender Vektor zur konditionalen Genexpression in Eukaryonten bereitgestellt, enthaltend, in funktioneller Verbindung, auf einem DNA-Molekül oder auf zumindest zwei getrennten DNA-Molekülen, zumindest eine Polynukleotidsequenz, die für einen mit Tetrazyklin oder seinen Derivaten oder Analogen regulierbaren Transaktivator (tTA) kodiert, wobei der tTA einen prokaryontischen Tet-Repressor in funktioneller Verbindung mit einem Polypeptid umfaßt, das direkt oder indirekt die Transkription in eukaryontischen Zellen aktiviert, einen tTA-responsiblen Promotor, der die tet-Operator-Sequenz (tetO) enthält, an die tTA binden kann, eine Polynukleotidsequenz, die zumindest für eine α-Amanitin-resistente Form der großen Untereinheit der RNA-Polymerase II kodiert, eine Polyadenylierungsstelle (PAA), eine Polynukleotidsequenz für zumindest einen selektierbaren Marker, und Polynukleotidsequenzen, die eine episomale Replikation des Vektors oder der Vektoren zumindest in Eukaryonten bewirken.

In der einen Ausführungsform der Erfindung sind die obenbeschriebenen Elemente des Vektors auf einem einzigen Vektormolekül vereinigt. Es ist jedoch erfindungsgemäß auch möglich, diese Elemente auf mehrere, bevorzugt auf zwei episomal replizierende Vektoren zu verteilen. In einer Ausführungsform der Erfindung sind die im Anspruch 1 näher gekennzeichneten Elemente des Vektorsystems auf zwei episomal replizierende Vektoren aufgeteilt, wobei (a) Vektor I tTA, eine Polynukleotidsequenz für zumindest einen selektierbaren Marker und Polynukleotidsequenzen, die eine episomale Replikation des Vektors zumindest in Eukaryonten bewirken, umfaßt, und (b) Vektor II den tetA-responsiblen Promotor, eine α-Amanitin-resistente Form der großen Untereinheit der RNA-Polymerase II, eine Polynukleotidsequenz für zumindest einen selektierbaren Marker und Polynukleotidsequenzen, die eine episomale Replikation des Vektors zumindest in Eukaryonten bewirken, umfaßt.

Der Tet-Repressor von tTA kann bevorzugt ein von Tn10-stammender Tet-Repressor sein.

Das Polypeptid von tTA, das die Transkription in eukaryontischen Zellen direkt oder indirekt aktiviert, kann aus dem Herpes simplex-Virus-Virionprotein 16 stammen.

Es kann aus mindestens einem Element der Gruppe saurer, prolinreicher, serin/threonin-reicher und glutaminreicher Transkriptions-Aktivatorpolypeptide ausgewählt sein.

Das Polypeptid von tTA, das die Transkription in eukaryontischen Zellen direkt oder indirekt aktiviert, kann eine Interaktionsdomäne sein, ausgewählt aus mindestens einem Element der Gruppe, bestehend aus einer Leucin-Zipper-Domäne, einer Helix-Loop-Helix-Domäne und einer Zinkfinger-Domäne. In einer Ausführungsform der Erfindung handelt es sich um eine Interaktionsdomäne aus einem TATA-Bindungsprotein.

Die für tTA kodierende Polynukleotidsequenz weist einen Promotor auf, der bevorzugt aus einem Element der Gruppe ausgewählt ist, bestehend aus CMV-, tk- und myc-Promotoren. Der durch Tetrazyklin regulierbare Transkriptionsaktivator ist somit ein Fusionsprotein (bezeichnet als tTA), das aus zwei Polypeptiden zusammengesetzt ist. Das erste Polypeptid ist ein tet-Repressor, der an tet-Operatorsequenzen binden kann. In einer Ausführungsform der Erfindung bindet er in Abwesenheit von Tetrazyklin, nicht jedoch in Anwesenheit von Tetrazyklin. In einer weiteren Ausführungsform der Erfindung bindet der Tet-Repressor an tet-Operatorsequenzen in Anwesenheit von Tetrazyklin, nicht dagegen in Abwesenheit von Tetrazyklin. Das zweite Polypeptid aktiviert direkt oder indirekt die Transkription in Eukaryontenzellen. Ein bevorzugtes Beispiel für das zweite Polypeptid ist die Transkriptionsaktivatordomäne des Herpes simplex-Virus-Virion-Proteins 16.

Zur Offenbarung des Tet-Repressor/Operator/Induktor-Systems wird vollinhaltlich auf die WO 94/29442, auf die Veröffentlichung von Gossen und Bujard in Proc. Natl. Acad. Sci., Band 89, S. 5547-5551 und auf die Veröffentlichung von Gossen et al. in Science, Band 268, S. 1766-1769 hingewiesen. Diese Veröffentlichungen werden vollinhaltlich in die vorliegende Anmeldung mit aufgenommen.

Die für tTA kodierende Polynukleotidsequenz steht bevorzugt unter Kontrolle eines konstitutiv aktiven Promotors, der bevorzugt ausgewählt wird aus einem Element der Gruppe, bestehend aus CMV-, tk- und c-myc-Promotoren, oder die tTA-kodierende Polynukleotidsequenz steht unter Kontrolle eines tTA-responsiblen Promotors (Autoregulation).

Der tTA-responsible Promotor besteht mindestens aus einer tet-Operatorsequenz und einem Promoter, bevorzugt einem Minimalpromotor, der die Transkription des Gens von Interesse initiiert. Unter einem "Minimalpromotor" ist eine Teilpromotorsequenz zu verstehen, die den Transkriptionsstart definiert, die aber selbst allein nicht ausreichend in der Lage ist, die Transkription wirksam zu initiieren. Die Aktivität derartiger Minimalpromotoren hängt von der Bindung von Aktivatoren an funktionell damit verbundene Bindungsstellen ab, beispielsweise von der Bindung des tetrazyklin-regulierten Transaktivators.

Der tTA responsible Promotor umfaßt zumindest eine Sequenz mit Promotorfunktion und eine Sequenz mit Operatorfunktion, an die tetR binden kann.

Der tTA-responsible Promotor enthält in einer bevorzugten Ausführungsform der Erfindung den TP-Promotor des Epstein-Barr-Virus (EBV).

In einer Ausführungsform der Erfindung umfaßt der tTA-responsible Promotor den TP-Promotor von EBV, bei dem das EBNA2-respon-sible Element durch die tet-Operator-Sequenz (tetO) ersetzt oder ergänzt wurde. Die Funktion des EBNA2-responsiblen Elements ist bekannt (vgl. beispielsweise Zimber-Strobl 1993, 1994).

Der tTa-responsible Promotor liegt in Verbindung mit der tet-Operator-Sequenz (tetO) vor. tetO liegt bevorzugt in multipler Kopienanzahl vor, insbesondere bevorzugt in einer 7-fachen Kopie.

Der tTA-responsible Promotor umfaßt somit die tet-Operator-Sequenz und einen Promotor zur Initiation der Transkription des Gens von Interesse, wobei es sich hier bevorzugt um einen Minimalpromotor handelt.

Der erfindungsgemäße Vektor enthält zumindest einen selektierbaren Marker, der an sich aus dem Stand der Technik bekannt ist. Beispiele hierfür sind Polynukleotidsequenzen, die für ein Tk-Protein, ein Neomycin-Protein oder ein Hygromycin-Protein kodieren.

Der erfindungsgemäße Vektor enthält bevorzugt solche Polynukleotidsequenzen, die für die episomale Replikation des Vektors in Eukaryonten verantwortlich sind. Die erfindungsgemäßen Vektorsysteme enthalten aber auch solche Polynukleotidsequenzen, die eine Replikation des Vektors in Prokaryonten ermöglichen, so daß es sich bei dem erfindungsgemäßen Vektorsystem um einen Shuttle-Vektor handelt.

Die für eine episomale Replikation des erfindungsgemäßen Vektorsystems zumindest in Eukaryonten verantwortlichen Polynukleotidsequenzen umfassen in einer Ausführungsform der Erfindung den Replikationsursprung (ori) von EBV (oriP) oder von BPV (Bovines Papilloma-Virus). Zur Aufrechterhaltung der episomalen Replikation bei Verwendung des EBV-Systems ist neben dem oriP weiterhin eine Polynukleotidsequenz notwendig, die für EBNA1 kodiert.

### Verpackung: Verpackbarkeit der Vektoren in EBV Partikel

Das Genom des EBV liegt in infizierten Zellen grundsätzlich in zwei Zuständen vor: dem Zustand der Latenz und der Virusproduktion (lytischer Zustand). Der letztere Zustand ist gekennzeichnet durch die Vervielfältigung der viralen DNA (Replikation) und die Expression einer Vielzahl von viralen Genprodukten, wie z.B. der Strukturproteine der viralen Partikel. Die lytische Replikation der viralen DNA beginnt an definierten DNA-Strukturen: Den sogenannten "lytic origins of DNA replication", die dupliziert im Genom des Virus vorliegen (orilyt) (Hammerschmidt et al., 1988). Die Amplifikation der viralen DNA durch Replikation ist Voraussetzung für eine effiziente Verpackung. Weiterhin werden für die Verpackung in virale Partikel noch die Enden der viralen DNA, die sogenannten "terminalen Repetitionen" (TR) benötigt (Hammerschmidt et al., 1989). Die Erweiterung der erfindungsgemäßen Vektoren um diese beiden Strukturen (orilyt und TR) erlaubt die Verpackung in virale Partikel. Nach Transfer der Vektoren in eine EBV positive Zelle und der Induktion des lytischen Zyklus werden diese in Form von Multimeren in infektiöse Partikel verpackt. Diese Partikel können dann zur Infektion von den EBV-Rezeptor tragenden Zellen zum Einsatz kommen.

Dieses Vorgehen kann dann zum Einsatz kommen, wenn sich die gewünschten Zielzellen nicht mit den herkömmlichen biophysikalischen Methoden transfizieren lassen.

Die erfindungsgemäß definierten Vektoren können auch als Genkonstrukte bezeichnet werden. Der Ausdruck "Vektor" umfaßt sowohl einen einzelnen Vektor als auch eine Kombination von mindestens zwei Vektoren, auf die die erfindungsgemäß notwendigen Polynukleotidsequenzen verteilt vorliegen.

Die Erfindung verwendet in einer bevorzugten Ausführungsform das System der tetrazyklin-abhängigen Genregulation zusammen mit EBV-Vektoren, die eine episomale Replikation gestatten.

Das Genom des EBV liegt in der infizierten Zelle in der Regel episomal mit einer Kopienzahl zwischen 10 und 100 vor. Im Zustand der Latenz geht die Replikation des viralen Genoms von einem Replikationsursprung (oriP) aus (Ytes et al., 1984). Zur Aufrechterhaltung der episomalen Replikation ist weiterhin die Bindung des EBNA1-Proteins an den oriP (Yates et al., 1985) notwendig. Die von EBV abgeleiteten Vektoren bestehen aus beispielsweise pBR-Sequenzen oder anderen Plasmidsequenzen, oriP, einer Expressionskassette für EBNA1 und einem Selektionsmarker für eukaryonte Zellen (z.B. das Hygromyzinresistenzgen). Diese Vektoren besitzen darüber hinaus noch die Kapazität für 20 bis 30 kb weitere Fremd-Sequenzen. Konstrukte, die auf diesen Vektoren basieren, werden in der Zelle: (i) auch ohne Selektion sehr viel besser "zurückgehalten" (Middleton and Sugden, 1994), (ii) erlauben eine geregelte Expression frei von Positionseffekten und (iii) besitzen ein wesentlich vermindertes Risiko der Insertionsmutagenese. Die Effizienz, mit diesen Vektoren stabil transfizierte Zellen zu erhalten, ist im Vergleich zu reinen Plasmidvektoren wesentlich höher. Wir konnten zeigen, daß mit Hilfe dieser Vektoren transfizierte regulierbare Genkonstrukte über einen langen Zeitraum ihre Indinzierbarkeit behielten (Polack et al., 1992; Bouvier et al., 1993).

In einer bevorzugten Ausführungsgorm der Erfindung wird das erfindungsgemäße Vektorsystem in EBV-positive Zellen eingebracht. EBV-positive Zellen exprimieren diejenigen Proteine, die für eine episomale Replikation des erfindungsgemäßen Vektorsystems notwendig sind. Es ist in diesem Fall nicht notwendig, beispielsweise daß ESNA1-Protein durch das erfindungsgemäße Vektorsystem exprimieren zu lassen.

Die erfindungsgemäß verwendeten, in der Zielzelle episomal replizierenden Vektoren vermeiden die aus dem Stand der Technik bekannten Schwierigkeiten, die mit einer stabilen Expression mit integrierenden Vektoren verbunden sind. Um dies für die Expression der tetrazyklin-abhängigen Transaktivatoren zu zeigen, wurde zunächst die Expressionskassette für tTA (konstitutiver Promotor, Polynukleotidsequenz, die für ein Gen kodiert (hier: tTA) und Polyadenylierungsstelle) unter der Kontrolle des Cytomegalie-Virus "immediate early gene"-Promotors (CMV) bzw. des Thymidinkinase (tk)-Promotors in vom Epstein-Barr-Virus (EBV) abgeleiteten Vektor pHEBOPL einkloniert. pHEBOPL ist ein Derivat (Polack et al., 1991) des von Sugden et al. (1985) beschriebenen Vektors.

### Konstruktionsbeschreibung:

### BC89-Konstruktionsbeschreibung

Der CMV-Expressionsvektor für tTA pUHD15-1 (Gossen und Bujard, 1992) wurde als XhoI und HpaI-Fragment (Sequenzposition 1 bis 1924) in die Restriktionsenzymspaltstellen SalI und NaeI (Sequenzposition 47 bis 79) des Vektors pHEBOPL (Erstbeschreibung: (Polack et al., 1991) einkloniert.

### BC90-Konstruktionsbeschreibung

Der tk-Expressionsvektor für tTA pUHD15-20 (Gossen und Bujard, 1992) wurde als XhoI und Hpal-Fragment (Sequenzposition 1 bis 1924) in die Restriktionsenzymspaltstellen SalI und NaeI (Sequenzposition 47 bis 79) des Vektors pHEBOPL einkloniert (BC90).

Die Expressionsplasmide BC89 und BC90 wurden zunächst transient in Raji-Zellen zusammen mit pUHC13-3 (vgl. Gossen und Bujard, 1992) transfiziert. Die Transfektionsansätze wurden anschließend für 48 Stunden mit und ohne Tetrazyklin (TC) 1 µg/ml behandelt. Die Messung der Luziferaseaktivität in Extrakten von diesen Zellen zeigte, daß die Plasmide BC89 und BC90 ebenso tTA exprimierten wie die Ausgangsplasmide pUHD15-1 und pUHD15-20 (Daten nicht gezeigt).

Weiterhin wurde die Burkitt-Lymphom-Zellinie Raji mit den Plasmiden BC89 und BC90 transfiziert. Durch Selektion mit Hygromycin wurden mehrere Sublinien (A - D) etabliert.

Durch transiente Transfektion von pUHC13-3 (CMV-tetO7-LUC) in die so entstandenen Sublinien wurde die stabile Expression des Tc-abhängigen Transaktivators nachgewiesen (Abb. 1').

Dieses Experiment zeigt, daß in Lymphomzellen durch stabile Transfektion eines episomal replizierenden Expressionsvektors funktionelle Mengen von tTa exprimiert werden können. Die vier bzw. drei verschiedenen Transfektanten mit BC89 bzw. BC90 zeigen untereinander keine Unterschiede hinsichtlich der Basisaktivität des CMV-tetO7-Promotors oder dessen Induzierbarkeit. Weiterhin zeigt das Experiment, daß der tk-Promotor (BC90) keine ausreichende Expression des Transaktivators erlaubt. Durch Tc wird in diesen Zellen (BC90A-C) die Luziferase-Expression lediglich um den Faktor 2 moduliert im Gegensatz zum 60- bis 100-fachen Unterschied in den CMV-tTA tragenden Zellinien (BC89A-D). Das Experiment belegt weiterhin, daß die Basisaktivität (d.h. minus TC) des pUHC13-3-Konstruktes durch die Menge an tTA nicht wesentlich beeinflußt wird.

Der Promotor von CMV-tetO7-LUC (pUHC13-3) war im uninduzierten Zustand (ohne Tc) etwa ein Drittel so aktiv wie der Standardexpressionsvektor CMV-LTR-LUC (Abb. 2). Im induzierten Zustand war pUHC13-3 sogar ca. 90-fach stärker als CMV-LTR-LUC. Für funktionelle Analysen erschien die "Basalaktivität" sowie die maximale Expression wesentlich zu hoch. Der ebenfalls von Gossen und Bujard hergestellte tk-Promotor mit tetO7 (pT7t81) zeigte im Vergleich zu pUHC13-3 zwar eine geringere Basalaktivität, aber die Aktivierbarkeit war mit Faktor 26 am schlechtesten im Vergleich mit den anderen getesteten Promotoren (Abb. 3).

Ein bevorzugtes Ziel der vorliegenden Erfindung ist der Einsatz der erfindungsgemäß episomal replizierenden Vektorsysteme in B-Zellen.

Die beiden bisher getesteten Promotoren erschienen für den Gebrauch in B-Zellen ungeeignet. Es wurde daher der TP-Promotor des EBV als Basis für die Konstruktion eines Tc-regulierbaren Expressionsvektors gewählt. Dieser Promotor wird in EBV-positiven B-Zellen äußerst effizient durch den von EBV kodierten Transkriptionsfaktor EBNA2 kontrolliert (Zimber-Strobl et al., 1991). Daß das DNA-Element im TP-Promotor an das EBNA2 im Komplex mit RBPJK bindet, ist bekannt (Zimber-Strobl et al., 1993; Meitinger et al., 1994). Die Regulation der Promotoraktivität wird durch den DNA-bindenden, zellulären Transkriptionsfaktor RBPJK vermittelt (Zimber, Strobl et al., 1994). Da der TP-Promotor ohne EBNA2 vollkommen inaktiv ist und durch EBNA2 um mehrere Größenordnungen aktiviert werden kann, wurde das ENBA2-responsible Element in diesem Promotor durch das tetO7-Element aus pUHC13-3 ersetzt.

### CR276-Konstruktionsbeschreibung

Die EBNA2-responsible Stelle im TP-LUC-Promotorkonstrukt Ga38-14 wurde über die Restriktionsenzymschnittstellen NruI und BclI entfernt. Die überstehenden Enden wurden durch Behandlung mit Klenow-Polymerase aufgefüllt und anschließend das tet-Operon (tetO7) als SmaI-SmaI-Fragment aus dem Ursprungsklon (pUHC13-3) von Gossen und Bujard (1992) eingesetzt.

Das TPtetO7-Luc-Konstrukt CR276 wurde transient in die tTA-exprimierende Raji-Zellinie BC89C transfiziert (Abb. 3). Der Vergleich mit pUHC13-3 zeigt, daß die "Basalaktivität" um den Faktor 4 bis 5 und die maximale (induzierte) Aktivität um den Faktor 6 bis 7 niedriger war.

### Die "all in one"-Strategie

Das von Gossen und Bujard (1992) vorgeschlagene System zur konditionalen Genexpression sieht vor, daß zunächst eine den Transaktivator (tTA) produzierende Zellinie hergestellt wird. Die optimale Zellinie wird durch transiente Transfektion geeigneter Reportergenkonstrukte ermittelt. In einem zweiten Transfektionsansatz soll dann das GOI, erfindungsgemäß das Gen für die große Untereinheit der α-Amanitin-resistenten RNA-Polymerase II, unter der Kontrolle des tet-Operons in diese Zelle eingeführt werden. Durch Testung verschiedener Subklone soll die geeignete, das GOI konditional exprimierende Zellinie identifiziert werden. Dieses Vorgehen setzt voraus, daß es möglich ist, mit Hilfe integrierender Vektoren eine größere Anzahl stabiler Transfektanten einer bestimmten Zellinie herzustellen, die über längere Zeit konstante Mengen des tTA-Moleküls produzieren. Weiterhin muß das GOI-Konstrukt auf solche Weise in das Wirtsgenom integrieren, daß es über längere Zeit für tTA zugänglich ist.

Dieses Vorgehen scheitert jedoch nicht selten an der Transfizierbarkeit von bestimmten Zellen (z.B. B-Zellen bzw. von B-Zellen abgeleitete Tumorzellen). Ein weiteres Problem stellt die permanente stabile Expression von Fremdgenen dar. Es ist bekannt, daß integrierende Expressionsplasmide, selbst wenn sie über Retroviren in die Zelle eingebracht werden, in vielen Zelltypen nach einiger Zeit abgeschaltet werden (Xu et al., 1989).

Um das Problem der zweistufigen Transfektion zu umgehen, wurde daher ein Plasmid erzeugt, das episomal repliziert, das tTA exprimiert und das unter der Kontrolle des TP-tetO7-Promotors trägt. Mit Hilfe dieses Plasmids sollte es möglich sein, in einem Schritt eine konditionale Expression des Luziferasegens zu erreichen, welches durch das Gen für die große Untereinheit der α-Amanitin-resistenten RNA-Polymerase ersetzbar ist.

### BC315-Konstruktionsbeschreibung

Zunächst wurden der TP-tetO7-Promotor und Teile des Luziferasegens als XmnI-ClaI-Fragment in BC89 ebenfalls über XmnI und ClaI einkloniert (BC250). Aus BC250 wurde das Luziferasegen über BglII und ClaI entfernt und ein BglII-Linker ("New England Biolabs" #1O66) eingefügt (BC252). Das Luziferasegen wurde zusammen mit der SV40-Polyadenylierungsstelle als BamHI-BglII-Fragment aus BC311 in die BglII-Schnittstelle von BC252 einkloniert
(BC315A). Als Kontrollplasmid wurde zusätzlich der Klon BC315B erzeugt, der das Luziferasegen in umgekehrter Orientierung enthält.

Es zeigte sich eine 100-fache Aktivierbarkeit des TPtetO7-Promotors auf dem Konstrukt BC315A. Dieses Ergebnis zeigte, daß weder die Vektoranteile (oriP, Hygromyzinresistenzgen) noch die tTA-Expressionskassette die Induzierbarkeit dieses Promotors beeinträchtigen. Es wurden daher stabile Raji-Transfektanten mit dem Plasmid BC315A etabliert und die Luziferaseaktivität vor und nach Tc-Behandlung gemessen (Abb. 5).

Das in Abb. 5 gezeigte Experiment zeigt die folgenden wesentlichen Merkmale des vorgestellten Systems:
· Auch nach stabiler Transfektion und der damit verbundenen Ausbildung einer geordneten Chromatinstruktur des eingeführten Plasmids bleibt die Induzierbarkeit des Promotors erhalten. Die räumliche Nähe des CMV-Promotor/Enhancer-Bereichs, der die Expression des tTA-Moleküls gewährleistet, zum TPtetO7-Promotor führt nicht zu dessen unmodulierbarer konstitutiver Expression.
· Die vier unabhängig voneinander entstandenen Sublinien zeigen nur eine geringgradige Variation hinsichtlich der Basalaktivität und der Induzierbarkeit der Luziferaseaktivität.

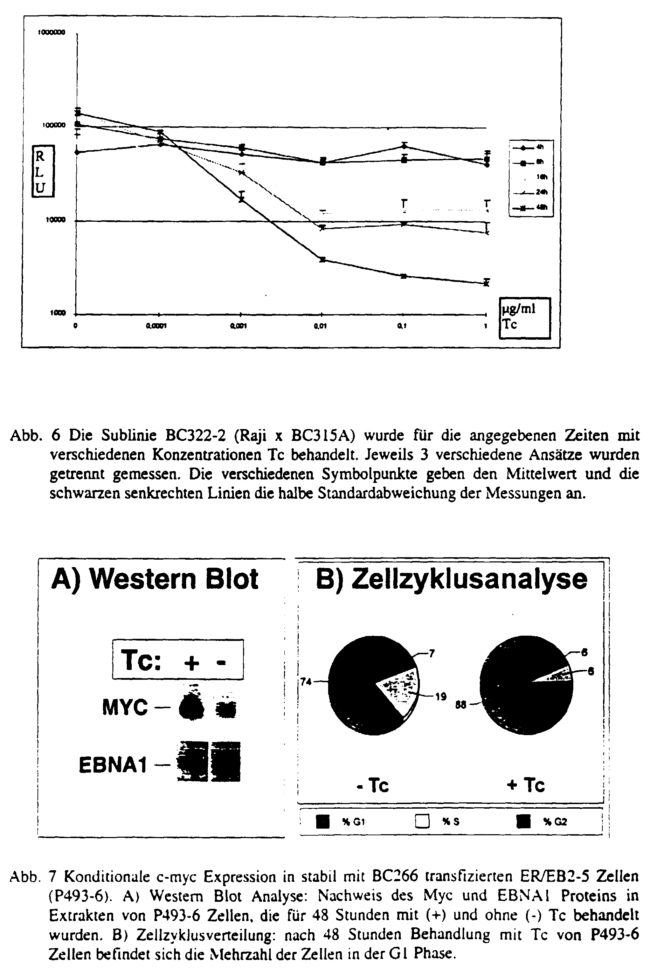

Die stabile Linie BC322-2 wurde hinsichtlich des zeitlichen Verlaufes und der Dosis-Wirkungsbeziehung genauer untersucht. Die Zellen wurden mit verschiedenen Konzentrationen an Tc behandelt und die Luziferaseaktivität nach 4, 8, 16, 24 und 48 Stunden bestimmt. Das Ergebnis des Experiments zeigt (Abb. 6), daß die maximale Wirkung nach 48 Stunden bei 1 µg/ml Tc erreicht wird. Da sowohl die Stabilität der Luziferase-RNA als auch des Proteins in dieses Experiment eingehen, kann über die tatsächliche Geschwindigkeit der Regulation der Promotoraktivität aufgrund dieses Experimentes keine Aussage getroffen werden. Eigene unveröffentlichte Beobachtungen der Stabilität des Luziferaseproteins haben gezeigt, daß die Halbwertszeit des Proteins mehrere Stunden beträgt. Es ist daher anzunehmen, daß die maximale Regulation der Promotoraktivität bereits innerhalb weniger Stunden nach Zugabe von Tc stattfindet.

Konditionale Expression des c-myc-Gens mit Hilfe des vorgestellten Systems. Anstelle des c-myc-Gens kann die α-Amanitinresistente RNA-Polymerase II eingesetzt werden.

### BC266-Konstruktionsbeschreibung

In die BglII-Schnittstelle des Vektors BC252 wurde ein Teil des 1. Introns, das 2. und 3. Exon, sowie die Polyadenylierungsstelle des humanen c-myc-Gens (Sequenzposition 4077 bis 8082 nach (Gazin et al., 1984) als BamHI-BamHI-Fragment einkloniert. Das c-myc-Gen stammt aus dem Burkitt-Lymphom BL60 (Polack et al., 1991). Die BamHI-Schnittstelle bei NheI (Position 4077) wurde mit Hilfe eines Linkers eingeführt (BC253). Die 3' von EcoRI (Sequenzposition von c-myc8082) gelegene BamHI-Schnittstelle stammt aus dem Polylinker des Vektors. Die konditionale Expression von Myc-Protein durch BC266 wurde durch transiente Transfektion und anschließende Western Blot-Analyse geprüft (Daten nicht gezeigt).

Um die konditionale Expression von Myc durch BC266 näher zu untersuchen, wurde die Zellinie ER/EB2-5 (Kempkes et al., 1995) stabil mit BC266 transfiziert. Die Zielzelle ER/EB2-5 wurde gewählt, da die c-myc-Expression in dieser Zellinie unter der Kontrolle einer konditionalen Mutante des EBNA2-Gens steht. Die konditionale EBNA2-Mutante (ER-EBNA2) wurde durch Fusion mit dem Östrogen bindenden Teil des humanen Östrogenrezeptors erzeugt. Die Funktion von ER-EBNA2 ist abhängig von der Anwesenheit von Östrogen im Zellkulturmedium. In dieser Zelle läßt sich die endogene Myc-Expression durch Östrogenentzug fast vollständig ausschalten (Kempkes et al., 1995).

Eine Sublinie dieser Transfektion (BC493-6) wurde näher untersucht. Die Myc-Expression in dieser Zellinie kann durch die Zugabe von Tc zum Zellkulturmedium stark moduliert werden (Abb. 7). Die Tc vermittelte Abschaltung von Myc wird von einem Proliferationsstop begleitet. Das in Abb. 7 gezeigte Experiment wurde in Abwesenheit von Östrogen, d.h. bei abgeschalteter EB-NA2-Funktion, durchgeführt. Das endogene c-myc-Gen ist in diesem Zustand fast nicht aktiv. Dieses Anwendungsbeispiel zeigt, daß mit Hilfe des Vektors BC252 eine konditionale Expression eines Nicht-Reportergens (c-myc) ereicht werden kann.

### Entwicklung von Vektoren, die durch Tetrazykline transkriptionell aktiviert werden

Gossen et al. (1995) haben eine Verbesserung ihres Systems vorgestellt. Der Tc-kontrollierte Transaktivator tTA wurde durch Mutagenese so verändert, daß er durch Tc nicht mehr reprimiert, sondern aktiviert wird. Dieses Molekül (rtTA) bindet nach Zugabe von Tc zum Zellkulturmedium an den Operator (tetO7).

### BC252rev Konstruktionsbeschreibung

In BC252 wurden über BspI407 und SplI große Teile des CMV-Promotors und des kodierenden Bereichs für tTA durch rtTA aus pUHD172-1 ebenfalls über BspI407 und SplI ausgetauscht. In das so entstandene Plasmid BC252rev wurde über BglII das Luziferasegen inklusive Polyadenylierungsstelle aus BC311 als BamHI-Fragment einkloniert (MS2a).

Mit MS2A wurden Raji-Zellen stabil transfiziert. Das Ergebnis der Testung von acht stabilen Transfektanten ist in Abb. 7 gezeigt.

Die beiden hier vorgestellten Vektorsysteme (BC252 und BC252rev) sind geeignet, in EBV-positiven Zellen eine konditionale Expression von beliebigen Genen zu erreichen. Für EBV-negative Zellen muß EBNA-1 entweder über ein zweites Plasmid in der Zelle exprimiert werden oder die EBNA1-Expressionskassette muß noch in die Vektoren BC252 und BX2S2rev eingefügt werden.

### Konditionale Expression der Raf-Mutante BXB

### Konstruktionsbeschreibung BC392

Die cDNA der Raf-Mutante BXB (Bruder et al., 1992) wurde als SfiI-SfiI-Fragment in die SfiI-Schnittstellen von BC364 einkloniert. BC364 ist ein Derivat von BC252, das zusätzlich das CAT-Gen (Chloramphenicolacetyltransferase) und eine Polyadenylierungsstelle enthält.

### Konditionale Expression von BXB in ER/EB2-5 Zellen

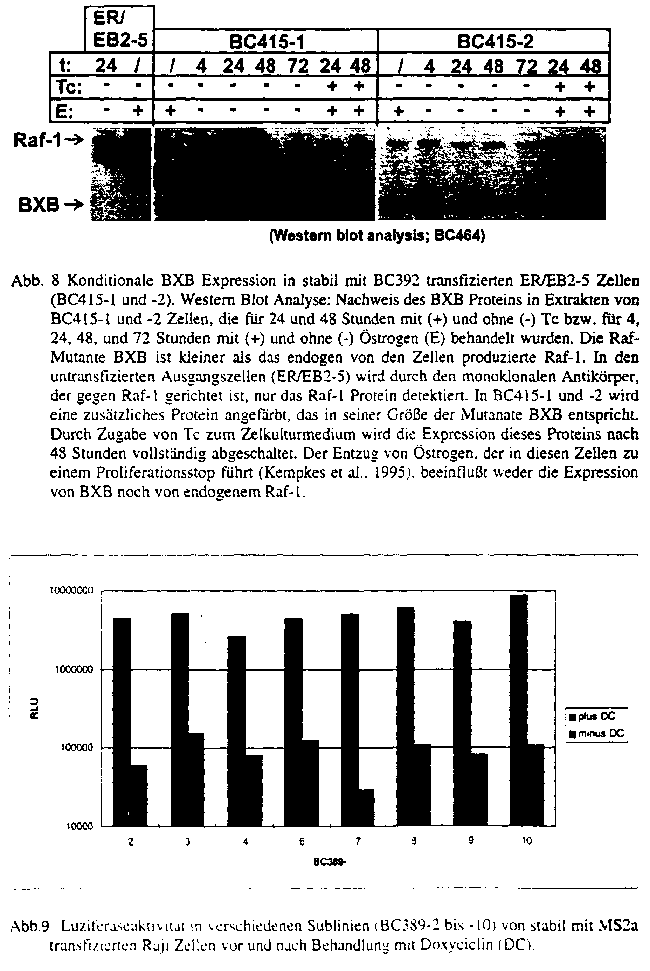

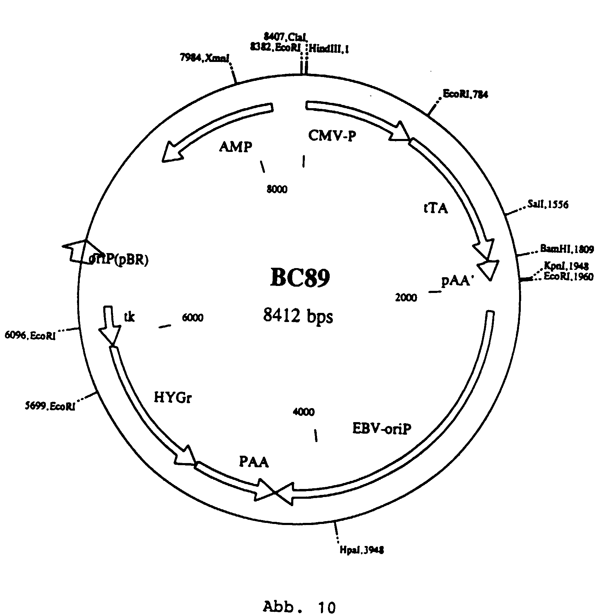

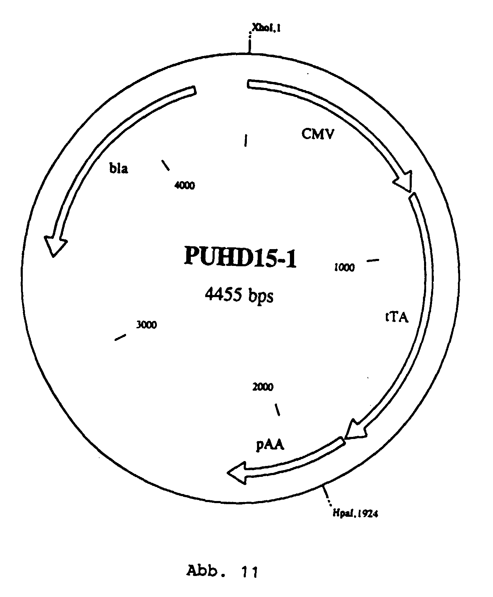

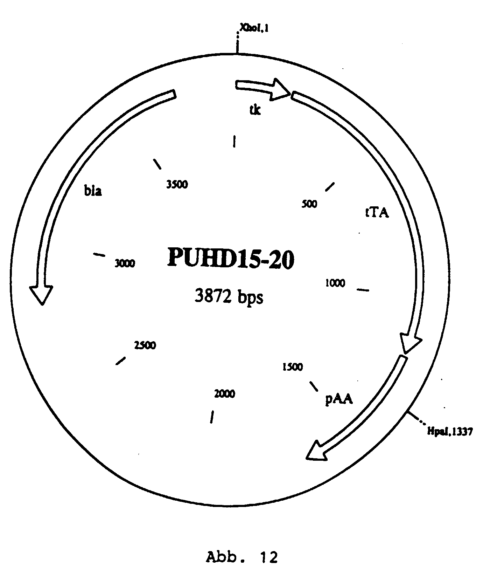

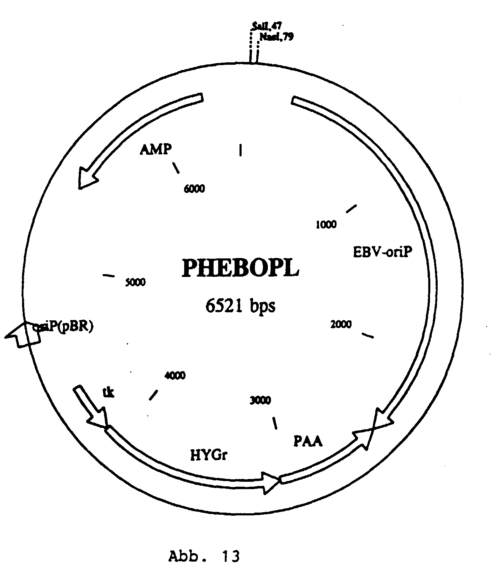

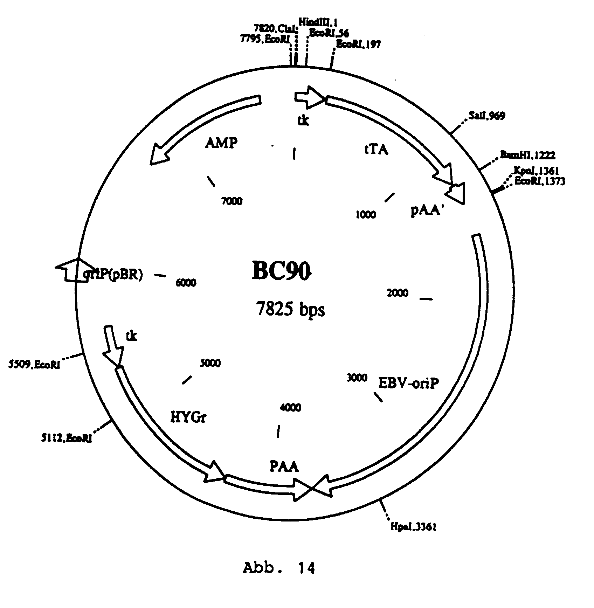

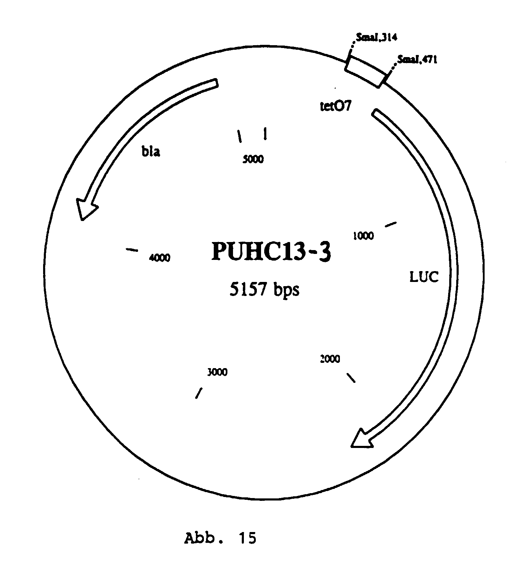

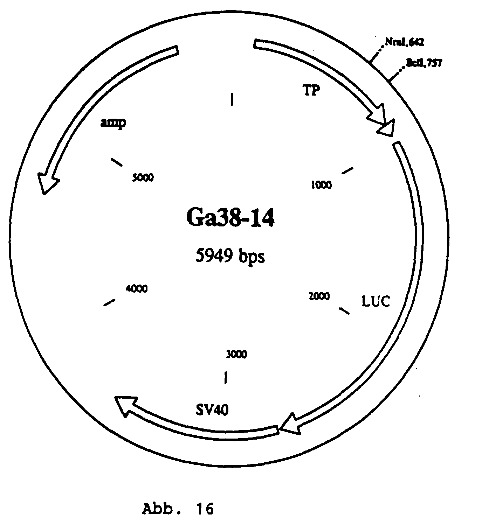

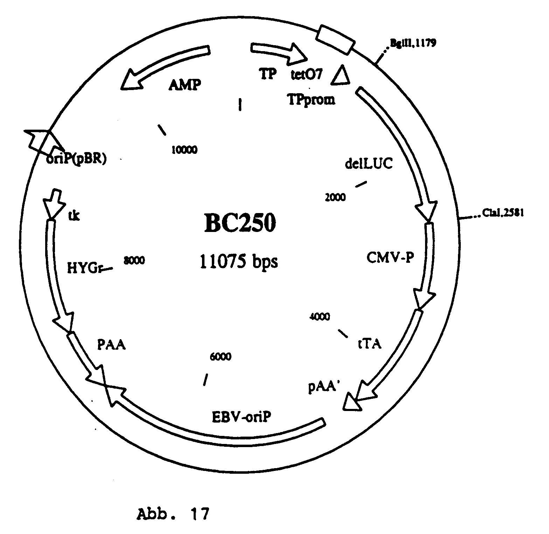

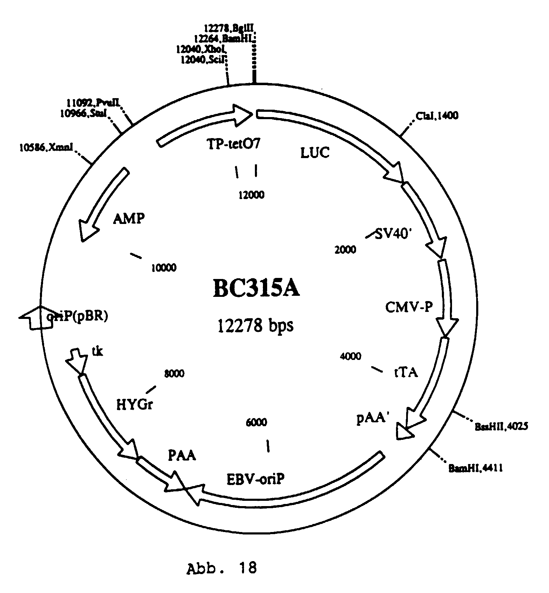

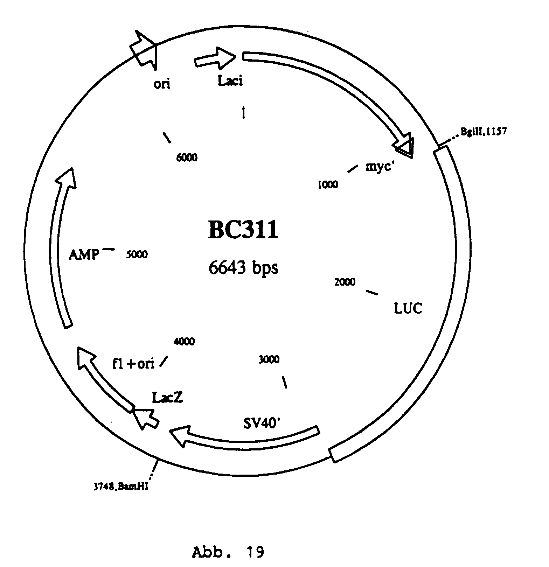

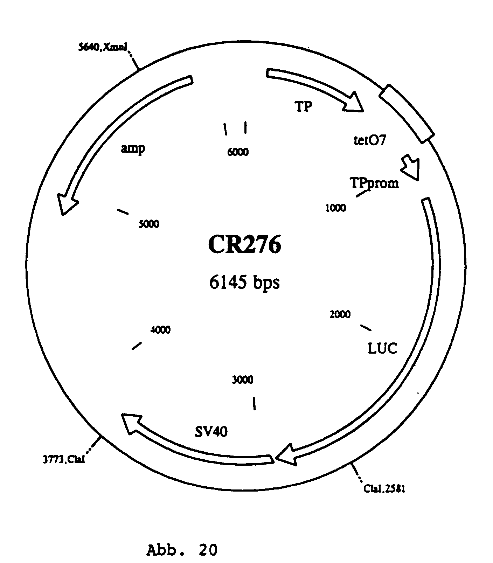

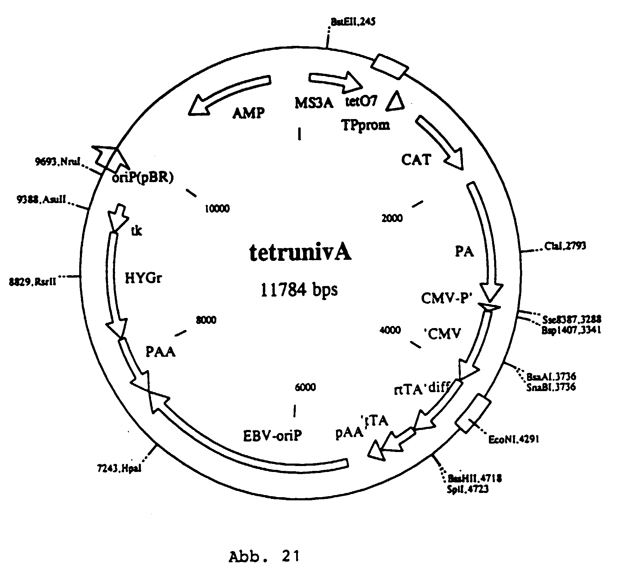

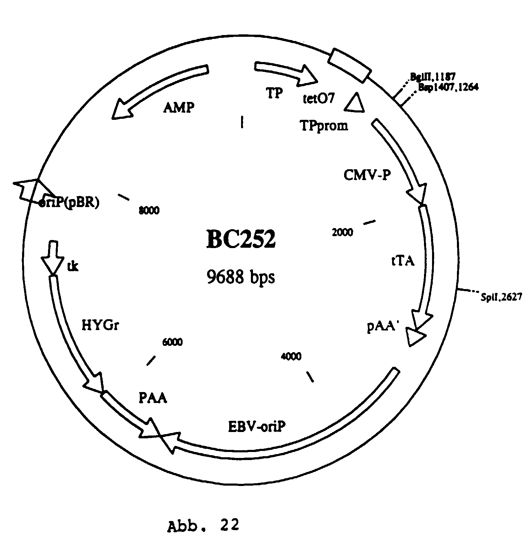

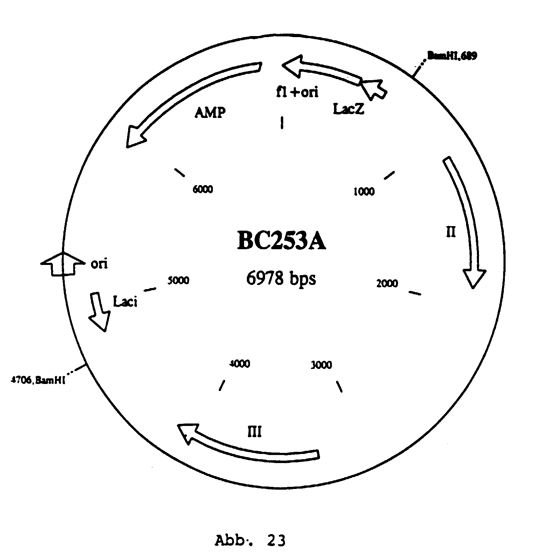

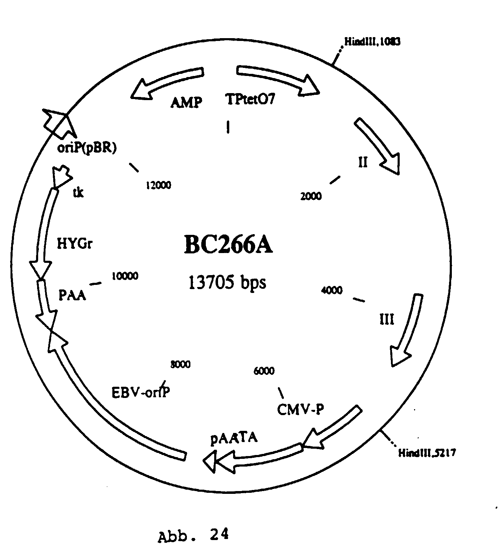

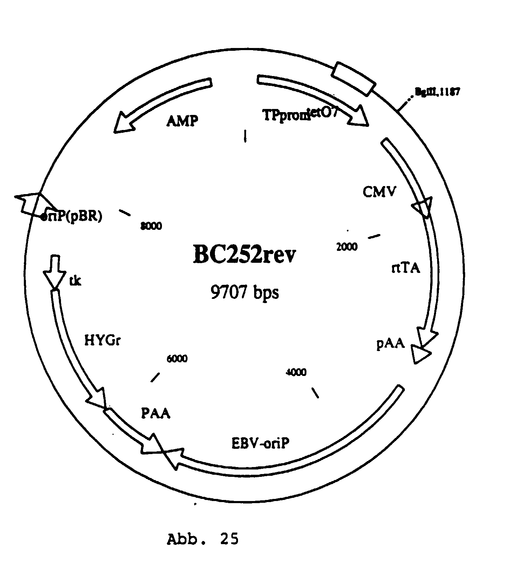

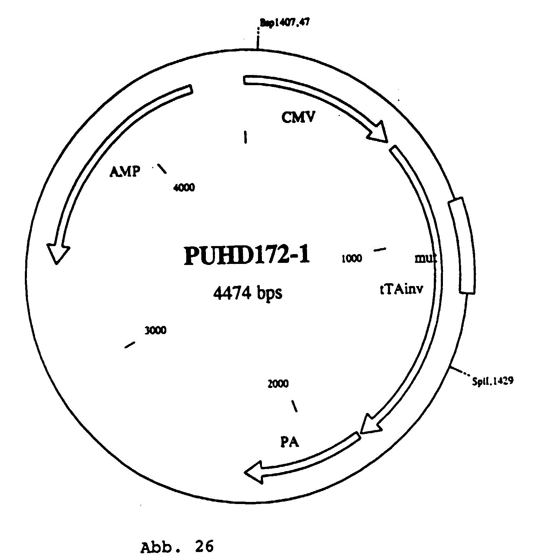

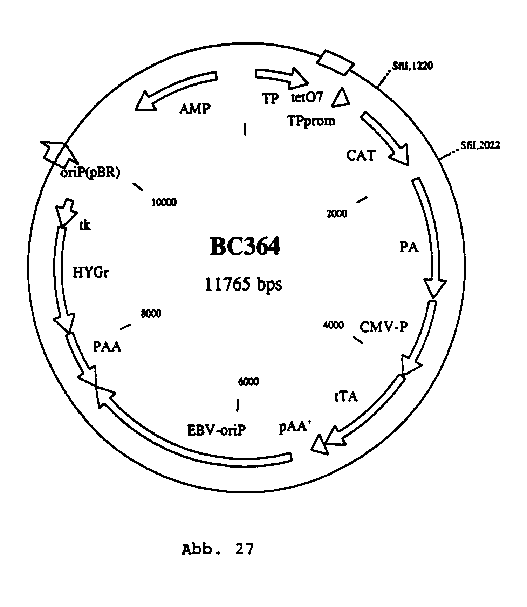

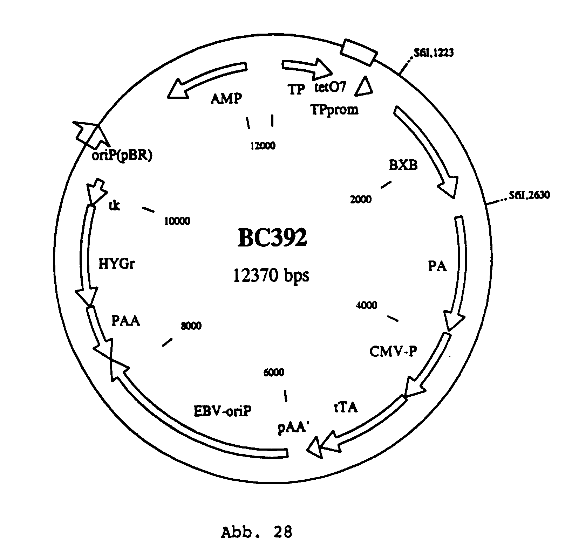

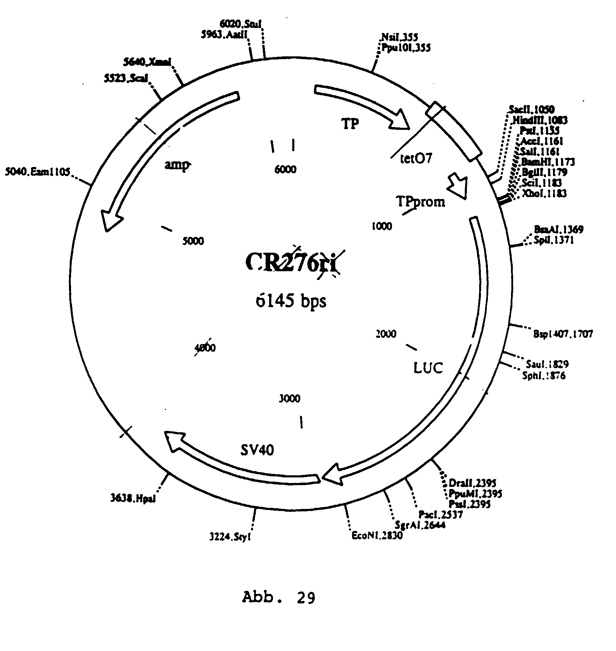

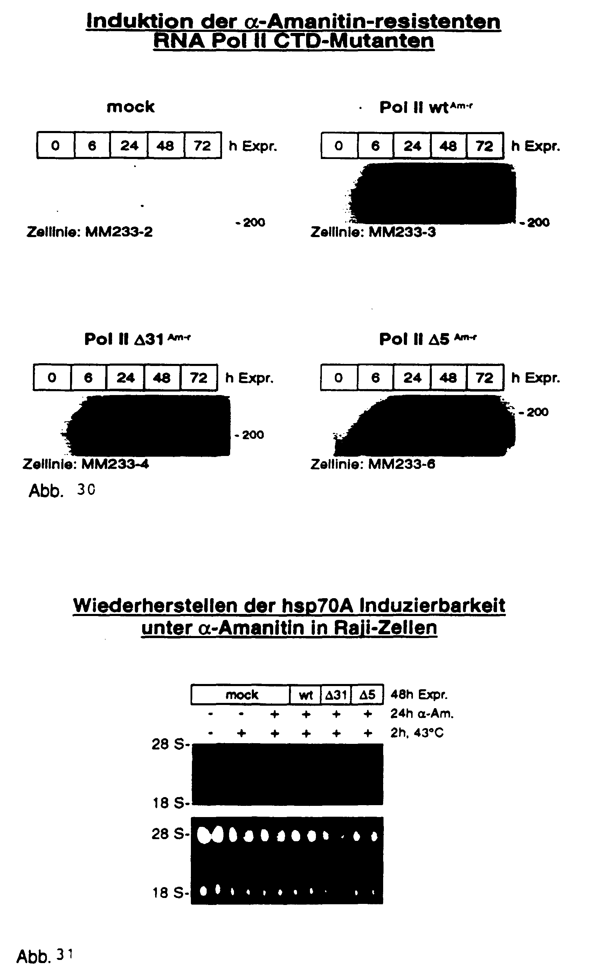

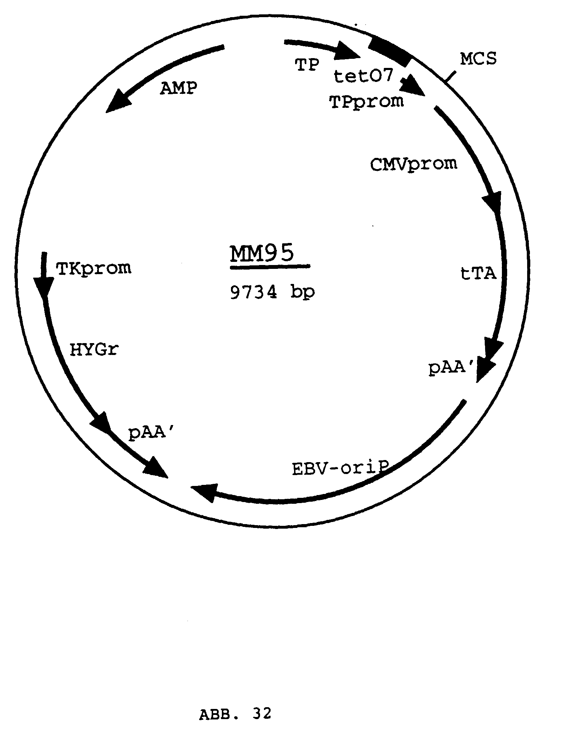

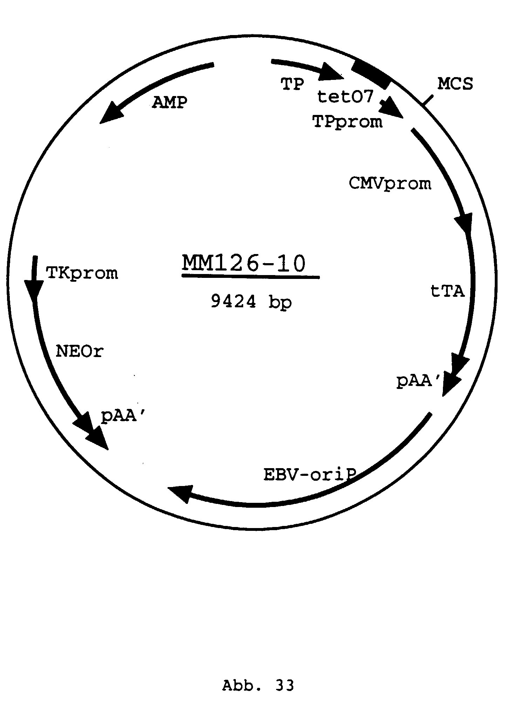

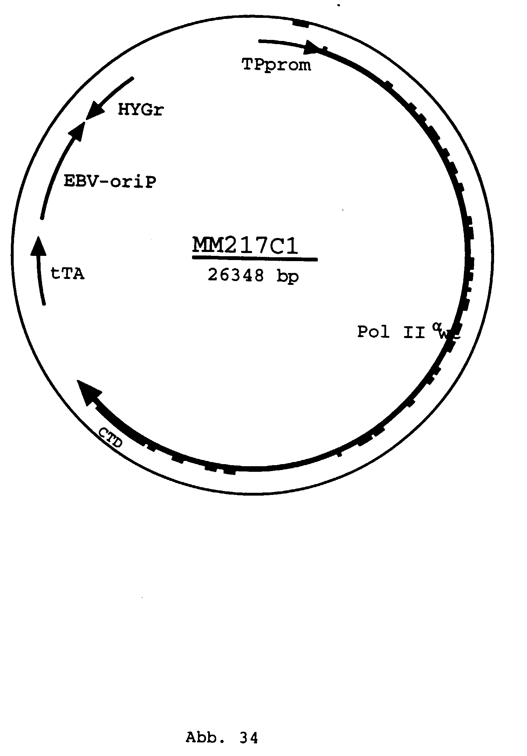

Das Konstrukt BC392 wurde in die Zellinie ER/EB2-5 transfiziert. Nach Selektion mit Hygromyzin wurden zwei stabile Linien etabliert; BC415-1 und BC415-2. Diese Zellinien wurden in An- und Abwesenheit von Östrogen und Tc für die in Abb. 8 angegebenen Zeiten kultiviert und Proteinextrakte gewonnen. Die Proteinextrakte wurden mit Hilfe der Western-Blot-Technik untersucht. Wie Abb. 8 zeigt, wird BXB in Abhängigkeit von Tc im Zellkulturmedium in BC 415-1 und -2 exprimiert wird.

Die ER/EB2-5-Zellen wachsen nur bei Anwesenheit von Östrogen im Zellkulturmedium (Kempkes et al., 1995). Östrogenentzug führt zu einem Proliferationsarrest. Die Expression des TPtetO7-Promotors wird nicht vom Proliferationszustand der Zellen beeinflußt. Der Vektor ermöglicht daher auch eine Expression in ruhenden Zellen.

Nachfolgend werden erfindungsgemäß besonders bevorzugte Ausgestaltungen des Vektors, der eine α-Amanitin resistente RNA-Polymerase II in konditional exprimierbarer Weise enthält, beschrieben:
- Der Tet-Repressor von tTA ist ein von Tn10-stammender Tet-Repressor.
- Das Polypeptid von tTA, das die Transkription in eukaryontischen Zellen direkt oder indirekt aktiviert, stammt aus dem Herpes simplex-Virus-Virionprotein 16.
- Das Polypeptid von tTA, das die Transkription in eukaryontischen Zellen direkt oder indirekt aktiviert, ist aus mindestens einem Element der Gruppe saurer, prolinreicher, serin/threonin-reicher und glutaminreicher Transkriptionsaktivatorpolypeptide ausgewählt.
- Das Polypeptid von tTA, das die Transkription in eukaryontischen Zellen direkt oder indirekt aktiviert, ist eine Interaktionsdomäne, ausgewählt aus mindestens einem Element der Gruppe, bestehend aus einer Leucin-Zipper-Domäne, einer Helix-Loop-Helix-Domäne und einer Zinkfinger-Domäne.
- Das Polypeptid von tTA, das die Transkription in eukaryontischen Zellen direkt oder indirekt aktiviert, ist eine Interaktionsdomäne aus einem TATA-Bindungsprotein.
- Die für tTA kodierende Polynukleotidsequenz steht unter Kontrolle eines konstitutiv aktiven Promotors, bevorzugt eines CMV-, tk- oder c-myc-Promotors oder Teilen hiervon, oder die tTA-kodierende Polynukleotidsequenz steht unter Kontrolle eines tTA-responsiblen Promotors.
- Der tTA-responsible Promotor enthält einen Minimalpromotor.
- Der tTA-responsible Promotor enthält zumindest Teile des TP-Promotors des EBV, des CMV-, tk- oder c-myc-Promotors.
- der tTA-responsible Promotor enthält den TP-Promotor von EBV, bei dem das EBNA2-responsible Element durch die tet-Operator-Sequenz (tetO) ersetzt oder ergänzt wurde.
   Der tTA-responsible Promotor ist ein Minimalpromotor in funktioneller Verbindung mit tetO.
- Der tTA-responsible Promotor ist ein CMV oder tk-Minimalpromotor oder ein TP-Minimalpromotor des EBV, je in funktioneller Verbindung mit tetO.
- Der tTA-responsible Promotor ist ein c-myc-P1-Promotor in funktioneller Verbindung mit tetO.
- Die tet-Operator-Sequenz (tetO) liegt in multipler Kopienanzahl vor, bevorzugt in sieben Kopien.
- Die Polynukleotidsequenz, die für einen selektierbaren Marker kodiert, ist das tk-Gen oder das Neomycin-Resistenzgen oder das Hygromycin-Resistenzgen.
- Die Polynukleotidsequenzen, die eine episomale Replikation des Vektors zumindest in Eukaryonten bewirken, enthalten den Replikationsursprung (ori) von EBV (oriP) oder von BP.
- Die Polynukleotidsequenz, die zumindest eine episomale Replikation des Vektors in Eukaryonten bewirkt, kodiert für EBNA1.
- Der Vektor enthält die Sequenzen für den lytischen Origin des EBV und die terminale Repeat-Struktur als Verpackungssequenz (TR).
- Der Vektor enthält Sequenzen für die Replikation in Bakterienzellen.
- tetO im tTA-responsiblen Promotor ist durch Bindung des Tet-Repressors positiv oder negativ regulierbar.

Die RNA Polymerase II und ihre Eigenschaften sind an sich bekannt. Beispielsweise wird hier auf die Veröffentlichung von Young, 1991 hingewiesen, auf die hier vollständig Bezug genommen wird.

Die Herstellung einer α-Amanitin-resistenten RNA Polymerase II ist Stand der Technik. Hier wird beispielsweise Bezug genommen auf die Veröffentlichungen von Bartolomei und Corden, 1987 und Bartolomei et al., 1988, auf die hier vollinhaltlich verwiesen wird.

Wie bereits oben beschrieben, enthält die große Untereinheit der RNA Polymerase II Repeatstrukturen. Diese Repeatstrukturen liegen in bevorzugten Ausführungsformen der vorliegenden Erfindung in mutierter Form vor. Unter Mutationen werden erfindungsgemäß insbesondere Insertionen, Deletionen und Substitutionen verstanden, die in den Repeatstrukturen lokalisiert sind. So können beispielsweise eine oder mehrere dieser Repeatstrukturen deletiert, durch ein anderes Repeatelement des gleichen Proteins oder eines anderen Proteins substituiert sein oder sie können durch die Insertion eines zusätzlichen Repeatelements oder mehrerer zusätzlicher Repeatelemente verändert, d.h. mutiert sein.

α-Amanitin gehört zum Stand der Technik. Derivate des α-Amanitins sind ebenfalls bekannt. Beispiele hierfür sind Aldoamanitin, Azoderivate von α-Amanitin, Etherderivate von α-Amanitin und Fetuinderivate von α-Amanitin. Nur beispielsweise wird auf die nachfolgenden Druckschriften Bezug genommen:

Faulstich et al., 1982; Baumann et al., 1993; Andres et al., 1986; Wieland et al., 1981; Baumann et al., 1994; Coulter and Greenleaf, 1982; Davies and Preston, 1981; Wieland et al., 1983; Preston et al., 1981; Mullersman and Preston, 1991; Kirchner and Faulstich, 1986; Zanotti et al., 1987; Mullersman et al., 1991; Fainlstich et al., 1981.

### Definitionen

In der obigen Beschreibung wird eine Anzahl von Ausdrücken der Rekombinanten DNA-Technologie verwendet. Nachfolgend werden einige Definitionen zur näheren Erläuterung dieser Ausdrücke ausgeführt:
**Klonierungsvektor**: Eine Plasmid- oder Phagen-DNA oder andere DNA-Sequenz, die in einer Wirtszelle autonom replizieren kann und die durch eine Anzahl von Restriktionsendonuklease-Erkennungsstellen charakterisiert ist. An diesen Stellen kann beispielsweise Fremd-DNA eingeführt werden. Der Klonierungsvektor kann weiterhin Markergene enthalten, um mit diesem Klonierungsvektor transformierte Zellen identifizieren zu können.
**Expressionsvektor:** Derartige Vektoren sind ähnlich wie Klonierungsvektoren aufgebaut, jedoch auch in der Lage, nach Überführung in eine Wirtszelle die Expression eines Fremdgens zu bewirken. Das in den Vektor einklonierte Fremdgen wird üblicherweise unter die Kontrolle von Kontrollsequenzen gestellt, beispielsweise Promotorsequenzen. Promotorsequenzen können entweder konstitutiv oder induzierbar sein.
**Eukaryontenzelle:** Eukaryontenzellen sind beliebige Zellen, in denen der Expressionsvektor bzw. der Klonierungsvektor eingebaut werden kann. Hierzu gehören beispielsweise Hefezellen, Pflanzenzellen, Säugerzellen, beispielsweise lymphoide Zellen, HeLa Zellen, NIH3T3 Zellen und embryonale Stammzellen.
**Rekombinate eukaryonten Wirtszelle:** Eine rekombinante eukaryonte Wirtszelle kann eine beliebige Eukaryontenzelle sein, die den erfindungsgemäßen Expressionsvektor enthält. Hierzu gehören auch solche Eukaryontenzellen, die genetisch verändert wurden, um die gewünschten Polynukleotidmoleküle auf dem Chromosom, Genom oder Episom des Organismus zu tragen. Rekombinante eukaryonte Wirtszellen sind in der Lage, die Proteine stabil oder transient zu exprimieren.
**Rekombinanter Vektor:** Jeder Klonierungs- oder Expressionsvektor, der die erfindungsgemäßen Polynukleotidmoleküle enthält.
**Wirtszelle:** Jede Prokaryonten- oder Eukaryontenzelle, die in der Lage ist, den erfindungsgemäßen Vektor aufzunehmen. Beispiele für derartige Wirtszellen sind angeführt in: Sambrook et al., Molecular Cloning: A Laboratory Manual, Second Edition, Cold Spring Harbor Laboratory, Cold Spring Harbor, N.Y. (1989).
**Promotor:** Eine DNA-Sequenz, die im allgemeinen als 5'-Abschnitt auf einem Gen beschrieben wird und proximal vom Startkodon lokalisiert ist. Die Transkription benachbarter Gene oder eines benachbarten Gens wird vom Promotorabschnitt initiiert. Falls es sich um einen initiierbaren Promotor handelt, wird die Transkriptionsrate in Anwesenheit des Induktionsmittels erhöht. Im Gegensatz dazu ist bei einem konstitutiven Promotor die Transkriptionsrate durch ein Induktionsmitttel nicht regulierbar.
**Gen:** Eine DNA-Sequenz, die die Information zur Expression eines Polypeptids oder Proteins enthält.
**Strukturgen:** Eine DNA-Sequenz, die in eine mRNA transkribiert wird und anschließend in eine Aminosäure-Sequenz translatiert wird, die für ein Polypeptid spezifisch ist.
**Polynukleotidmoleküle:** Ein Polynukleotidmolekül kann eine DNA oder RNA sein.
**Expression:** Durch die Expression wird ein Polypeptid von einem Strukturgen produziert. Die Expression umfaßt die Transkription des Gens zu einer mRNA und die Translation der mRNA in ein Polypeptid.
**Fragment:** Unter "Fragment" eines Moleküls wird eine DNA- oder Polypeptid-Teilsequenz verstanden.
**Tetrazyklinderivate:** Hierunter werden Verbindungen verstanden, die mit dem Tetrazyklin verwandt sind und die an den Tet-Repressor mit einer K_{α} von wenigstens etwa 10⁶ M⁻¹ binden. Bevorzugt bindet das Tetrazyklinderivate mit einer Affinität von etwa 10⁹ M⁻¹ oder darüber, beispielsweise 10¹¹ M⁻¹. Beispiele für Tetrazyklinderivate sind in den nachfolgenden Veröffentlichungen genannt: Hlavka und Boothe, "The Tetracyclines", Handbook of Experimental Pharmacology 78, R.K. Blackwood et al., (eds.), Springer-Verlag, Berlin-Neu York, 1985; L.A. Mitschef, "The Chemistry of the Tetracycline Antibiotics", Medicinal Research 9, Dekker, New York, 1978; Noyee Development Corporation, "Tetracycline Manufacturing Processes", Chemical Process Reviews, Park Ridge, N.J., 2 Bände, 1969; R.C. Evans, "The Technology of the Tetracyclines", Biochemical Reference Series 1, Quadrangle Press, New York, 1968; und H.F. Dowling, "Tetracycline", Antibiotics Monographs, No. 3, Medical Encyclopedia, New York, 1955.

### LITERATUR

Bouvier, G., Hergenhahn, M., Polack, A., Bornkamm, G.W., and Bartsch, H. (1993). Validation of two test systems tor detecting tumour promoters and EBV inducers: comparative responses of several agents in DR-CAT Roji cells end in human granulocytes. Carcinogenesis 14, 1573-1578.
Brinder, J.T., Heidecker & U.R. Rapp, Serum-, TPA-, and Ras-induced expression from Ap-1/Ets-driven promoters requires Raf-1 kinase. Genes Dev. 6: 545-56 (1992).
Gazin, C., Dinechin, S.D., Hampe, A., Masson, J.M., Martin, P., Stehelin, D., and Galibert, F. (1984). Nucleotide sequence of the human c-myc locus: provocative open reading e within the first exon. EMBO J. 3, 383-387.
Gossen, M., Freundlieb, S., Bender, G., Müller, G., Hlllen, W., and Bujard, H. (1995). Transcriptional activation by tetracyclines in mammalian cells. Science 268. 1766-1769.
Gossen, M. and Bujard. H. (1992). Tight control of gene expression in mammalian cells by tetracycline-responsive promoters. Proc. Nat. Acad. Sci. U.S.A. 89, 5547-5551.
Hamerschmidt, W. and Sugden, B. (1988). Identification and characterization of oriLyt, a lytic origin of DNA replication of Epstein-Barr Virus. Cell 55, 427-433.
Hammerschmidt, W. and Sugden, B. (1989). Genetic analysis of immortalizing functions of Epstein-Barr Virus in human B lymphocytes. Nature 340, 393-397.
Kempkes, B., Spitkovsky, D., Jansen-Dürr, P., Ellwart, J.W., Kremmer, E., Delecluse, H.-J., Rottenherger. C., Bornkamm. G.W., and Hammerschmidt. W. (1995). B-cell proliferation and induction of early G₁-regulating proteins by Epstein-Barr Virus mutants conditional for EBNA2. EMBO J. 14, 88-96.
Meitinger, C., Strobl, L.J., Marschall, G., Bornkamm, G.W., and zimber-Strobl, U. (1994). Crucial sequences within die Epstein-Borr vizus TP1 promoter for EBNA2-mediated transactivotion and interaction of EBNA2 with its responsive element. J. Virol. 68, 7497-7506.
Middleton, T. and Singden, B. (1994). Retention of plasmid DNA in mammalian cells is enhanced by binding of the Epstein-Barr virus replication protein EBNA1. J. Virol. 68, 4067-4071.
Polack, A., Strobl, L., Feederle, R., Schweizer, M., Koch, E., Eick, D., Wiegand, H., and Bornkamm, G.W. (1991). The intron enhancer of the immunoglobulin kappa gene activates c-myc but does not induce the Burkitt specific promoter shift. Oncogene 6, 2033-2040.
Polack, A., Laux, G., Hergenhahn, M., Kloz, U., Rösner, H., Hecker, E., and Bornkamm, G.W. (1992). Short-term assays for detection of conditional cancerogens I. Construction of DR-CAT Raji cells and some of their characteristics as tester cells. Int. J. Cancer 50, 611-616.
Sugden, B., Marsh, K., and Yates, J. (1985). A vector that replicates as a plasmid and can be efficiently selected in B-lymphoblasts transformed by Epstein-Barr virus. Mol. Cell Biol. 5, 410-413.
Xu, L., Yee, J.K., Wolff, J.A., and Friedmann, T. (1989). Factors affecting long-term stability of Moloney murine leukemia virus-based vectors. Virology 171, 331-341.
Yates, J., Warren, N., Reisman, D., and Sugden, B. (1984). A cis-acting element from the Epstein-Barr viral genome that permits stable replication of recombinant plasmids in latently infected cells. Proc. Natl. Acad. Sci. U.S.A. 81, 3806-3810.
Yates, J.L., Warren, N., and Sugden, B. (1985). Stable replication of plasmids derived from Epstein-Barr virus in various mammalian cells. Nature 313, 812-815.
Zimber-Strobl, U., Kremmer, E., Grosser, F., Marschall, G., Laux, G., and Bornkamm, G.W. (1993). The Epstein-Barr virus nuclear antigen 2 interacts with an EBNA2 responsive cis-element of the terminal protein 1 gene promoter. EMSO J. 12, 167-175.
Zimber-Strobl, U., Strobl, L.J., Meitinger, C., Hinrichs, R., Sakai, T., Furukawa, T., Honjo, T., and Bornkamm, G.W. (1994). Epstein-Barr virus nuclear antigen 2 exerts its trnnsactivating function through interaction with recombination signal binding protein RBP-J kappa, the homologue of Drosophila Suppressor of Hairless. EMBO J. 13, 4973-4982.
Zimber-Strobl, U., Suentzenich, K.-O., Laux, G., Eick, D., Cordier, M., Calender, A., Billaud, M., Lenoir, G.M., and Bornkamm, G.W. (1991). Epstein-Barr virus nuclear antigen 2 activates transcription of the terminal protein gene. J. Virol. 65, 415
Andres, R.Y., Frei, W., Gautschi, K., and Vonderschmitt, D.J. (1986) Radioimmunoassay for amatoxins by use of a rapid, 125I-tracer-based system. Clin Chem 32, 1751-5.
Bartolomei, M.S., and Corden, J.L. (1987). Localization of an alpha-amanitin resitance mutation in the gene encoding the largest subunit of mouse RNA polymerase II. Mo. Cell Biol 7, 586-94.
Bartolomei, M.S., Halden, N.F., Cullen, C.R., and Corden, J.L. (1988). Genetic analysis of the repetitive carboxyl-terminal domain of the largest subunit of mouse RNA polymerase II. Mol Cell Biol 8, 330-9.
Baumann, K., Munter, K., and Faulstich, H. (1993). Identification of structural features involved in binding of alpha-amanitin to a monoclonal antibody. Biochemistry 32, 4043-50.
Baumann,K., Zanotti,G., and Faulstich, H. (1994). A beta-turn in alpha-amanitin is the most important structural feature for binding to RNA-polymerase II and three monoclonal antibodies. Protein Sci 3, 750-6.
Coulter, D.E., and Grennleaf, A.L. (1982). Properties of mutationally altered RNA polymerase II of Drosophila, J Biol Chem 257, 1945-52.
Davis, M.T., and Preston, J.F.d. (1981). A conjugate of alpha-amanitin and monoclonal immunoglobulin G to Thy 1.2 antigen is selectively toxic to T lymphoma cells. Science 213, 1385-8.
Faulstich, H., Trischmann, H., Wieland, T., and Wulf, E. (1981). Ether derivatives of alpha-amanitin. Introduction of spacer moieties, lipophilic residues, and radioactive labels. Biochemistry 20, 6498-504.
Faulstich, H., Zobeley, S., and Trischmann, H. (1982). A rapid radioimmunoassay using a nylon support, for amatoxins from Amanita mushrooms. Toxicon 20, 913-24.
Kirchner, K., and Faustich, H. (1986). Purification of amatoxin-specific antibodies from rabbit sera by affinity chromatography, their characterization and use in toxicological studies. Toxicon 24, 273-83.
Mullersman, J.E., Bonetti, S.J., and Preston, J.F.d. (1991). Periodate oxidation products derived from methylated alpha-amanitin; evidence fro distinct aldehydic and non-aldehydic forms. Int J. Pept Protein Res 38, 409-16.
Mullersman, J. E., and Preston, J. F. d. (1991). Amatoxins bearing amino and carboxyl groups prepared by selective alteration of the aldehyde generated by periodate oxidation of methylated alpha-amanitin. Int. J Pept Protein Res 37, 544-51.
Preston, J. F., Hencin, R.S., and Gabbay, E.J. (1981). Preparation and characterization of an alpha-amanitin azo derivative with a free carboxyl group and its bovine serum albumin conjugate. Arch Biochem Biophys 209, 63-71.
Wieland, T., Gotzendorfer, C., Dabrowski, J., Lipscomb, W. N., and Shoham, G. (1983). Unexpected similarity of the structures of the weakly toxic amanitin (S)-sulfoxide and the highly toxic (R)-sulfoxide and sulfone as revealed by proton nuclear magnetic resonance and X-ray analysis. Biochemistry 22, 1264-71.
Wieland, T., Gotzendorfer, C., Zanotti, G., and Vaisius, A. C. (1981). The effect of the chemical nature of the side chains of amatoxins in the inhibition of eukaryotic RNA polymerase B. Eur J Biochem 117, 161-4.
Young, R. A. (1991). RNA polymerase II. Annu Rev Biochem 60, 689-715.
Zanotti, G., Mohringer, C., and Wieland, T. (1987). Synthesis of analogues of amaninamide, an amtoxin from the white Amanita virosa mushrooms. Int J Pept Protein Res 30, 450-9.

## Patentansprüche

1. Expressionsvektor für Eukaryontenzellen, der zumindest die nachfolgenden Elemente enthält:
(a) Das Gen für die große Untereinheit der RNA-Polymerase II oder mutierten Formen davon, wobei die RNA-Polymerase II oder die mutierten Formen davon gegen α-Amanitin oder einem Derivat hiervon resistent sind;
(b) Nukleotidsequenzen, die eine konditionale Regulation der Expression der großen Untereinheit des α-Amanitin-resistenten RNA-Polymerase II-Gens oder der mutierten Formen ermöglichen;
(c) Nukleotidsequenzen zur Selektion des Vektors in Prokaryonten- und/oder Einkaryonten-Zellen;
(d) Nukleotidsequenzen zur Replikation des Vektors in Prokaryonten-Zellen und/oder Einkaryonten-Zellen;
(e) eine Polyadenylierungsstelle (PAA).

2. Expressionsvektor nach Anspruch 1,
dadurch gekennzeichnet,
daß die mutierte Form der großen Untereinheit des α-Amanitin-resistenten RNA-Polymerase II-Gens eine Deletion, Insertion, und/oder Substitution in der carboxy-terminalen Domäne (CTD) der großen Untereinheit aufweist und/oder daß die konditionale Regulation durch eine durch Tetrazyklin oder seine Derivate regulierbare Polynukleotidsequenz erfolgt.

3. Expressionsvektor nach Anspruch 1 oder 2,
dadurch gekennzeichnet,
daß die Nukleotidsequenzen, die eine konditionale Regulation der großen Untereinheit des RNA-Polymerase II-Gens ermöglichen, nachfolgendes umfassen:
- eine Polynukleotidsequenz, die für einen mit Tetrazyklin oder seinen Derivaten oder Analogen regulierbaren Transaktivator (tTA) kodiert, wobei der tTA einen prokaryontischen Tet-Repressor in funktioneller Verbindung mit einem Polypeptid umfaßt, das direkt oder indirekt die Transkription in eukaryontischen Zellen aktiviert,
- einen tTA-responsiblen Promotor, der die Tet-Operator-Sequenz (TetO) enthält, an die tTA binden kann.

4. Expressionsvektor nach einem oder mehreren der vorhergehenden Ansprüchen,
dadurch gekennzeichnet,
daß er weiterhin Polynukleotidsequenzen aufweist, die eine episomale Replikation des Vektors bewirken und er in das DNA-Genom der Eukaryontenzelle nicht integriert.

5. Expressionsvektor nach einem oder mehreren der vorhergehenden Ansprüche,
dadurch gekennzeichnet,
daß die Elemente des Expressionsvektors auf zumindest zwei getrennten Vektoren lokalisiert sind.

6. Vektor nach Anspruch 5,
dadurch gekennzeichnet, daß
(a) der Vektor I tTA, eine Polynukleotidsequenz für zumindest einen selektierbaren Marker und Polynukleotidsequenzen, die eine episomale Replikation des Vektors zumindest in Eukaryonten bewirken, umfaßt, und
(b) Vektor II den tTA-responsiblen Promotor, das Gen für die große Untereinheit der RNA-Polymerase II, eine Polynukleotidsequenz für zumindest einen selektierbaren Marker und Polyninkleotidsequenzen, die eine episomale Replikation des Vektors zumindest in Eukaryonten bewirken, umfaßt.

7. Prokaryontenzelle oder Eukaryontenzelle, enthaltend einen Expressionsvektor nach einem oder mehreren der vorhergehenden Ansprüche.

8. Verwendung eines Expressionsvektors nach einem oder mehreren der vorhergehenden Ansprüche 1-7 für Untersuchungen über die Funktionalität einzelner Protein-Domänen der RNA-Polymerase II in Bezug auf von der RNA-Polymerase II transkribierte Gene.

9. Verwendung nach Anspruch 8 für Untersuchungen über die Funktionalität der carboxy-terminalen Domäne (CTD) der großen Untereinheit des RNA-Polymerase II-Gens in Bezug auf von der RNA-Polymerase II transkribierte Gene.

10. Verwendung des Expressionsvektors nach Anspruch 8 und 9 zur Untersuchung der Funktion der RNA-Polymerase II in der Transkription, Signalübertragung und Genregulation.

11. Verfahren zur konditionalen, stabilen Expression von α-Amanitin-resistenter RNA-Polymerase II mit den nachfolgenden Schritten:
(a) Einführen eines Expressionsvektors noch einem oder mehreren der Ansprüche 1-6 in eine Eukaryontenzelle;
(b) Selektion auf den Vektor enthaltende Zellen in Abwesenheit von α-Amanitin und Inhibierung der Expression des Gens für die große Untereinheit der α-Amanitin-resistenten RNA-Polymerase II;
(c) Induktion der Expression des Gens für die große Untereinheit der α-Amanitin-resistenten RNA-Polymerase II;
(d) Zugabe von α-Amanitin zur Blockade der Expression der endogenen, nicht α-Amanitin-resistenten RNA-Polymerase II.

12. Verfahren nach Anspruch 11,
dadurch gekennzeichnet,
daß der Vektor durch Transfektion oder Infektion eingeführt wird.

13. Verfahren nach Anspruch 11,
dadurch gekennzeichnet,
daß die Inhibierung der Expression im Schritt (b) durch Zugabe von Tetrazyklin oder seinen Derivaten oder Analogen erfolgt.

14. Verfahren nach Anspruch 11,
dadurch gekennzeichnet,
daß die Induktion der Expression im Schritt (c) durch Entfernen des Tetrazyklins oder seiner Derivate oder Analoge erfolgt.
